(19) Europäisches Patentamt
European Patent Office
Office européen des brevets

(11) Veröffentlichungsnummer: **0 003 360**
**B1**

# (12) EUROPÄISCHE PATENTSCHRIFT

(45) Veröffentlichungstag der Patentschrift: **30.12.81**

(51) Int. Cl.³: **C 07 D 417/12,** A 61 K 31/54 // (C07D417/12, 311/22, 279/02)

(21) Anmeldenummer: **79100226.4**

(22) Anmeldetag: **26.01.79**

(54) **Neue Azathianaphthalinderivate, Verfahren zu ihrer Herstellung, diese enthaltende pharmazeutische Präparate und ihre Verwendung.**

(30) Priorität: **30.01.78 CH 993/78**

(43) Veröffentlichungstag der Anmeldung: **08.08.79 Patentblatt 79/16**

(45) Bekanntmachung des Hinweises auf die Patenterteilung: **30.12.81 Patentblatt 81/52**

(84) Benannte Vertragsstaaten: **BE CH DE FR IT LU NL SE**

(56) Entgegenhaltungen:
**US-A-3 591 584**

**JOURNAL OF MEDICINAL CHEMISTRY, Band 16(1), Januar 1973, Washington, D.C. USA HAROLD ZINNES et al.: «1,2-Benzothiazines 6,3-Carbamoyl-4-Hydroxy-2H-1,2-benzothiazine 1,1-Dioxides as Anti inflammatory agents», Seiten 44-48**

(73) Patentinhaber: **CIBA-GEIGY AG, Patentabteilung Postfach, CH-4002 Basel (CH)**

(72) Erfinder: **Sele, Alex, Langmattstrasse 14, CH-4132 Muttenz (CH)**
Erfinder: **Ferrini, Pier Giorgio, Dr., Im Rehwechsel 22, CH-4102 Binningen (CH)**
Erfinder: **Haas, Georges, Dr., Im Rehwechsel 24, CH-4102 Binningen (CH)**
Erfinder: **Jaeggi, Knut A., Dr., General Guisan-Strasse 44, CH-4054 Basel (CH)**
Erfinder: **Rossi, Alberto, Dr., Bündtenweg 30, CH-4104 Oberwil (CH)**

(74) Vertreter: **Zumstein sen., Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Neue Azathianaphthalinderivate, Verfahren zu ihrer Herstellung, diese enthaltende pharmazeutische Präparate und ihre Verwendung

Die Erfindung betrifft neue Azathianaphthalinderivate, insbesondere substituierte 3,4-Dihydro-2H-1,2-benzothiazin-1,1-dioxyde der Formel I

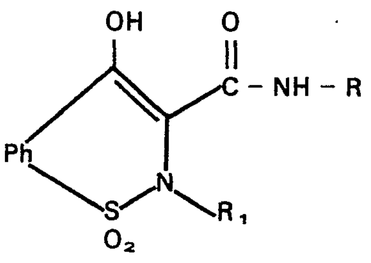

(I)

worin Ph einen gegebenenfalls durch Niederalkyl, Niederalkoxy, Niederalkanoyl, Halogen, Nitro und/oder Trifluormethyl substituierten 1,2-Phenylenrest bedeutet, R einen gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino oder an zwei benachbarten Kohlenstoffatomen durch Niederalkylen oder Niederalkylendioxy substituierten Benzopyronrest bedeutet, und $R_1$ Wasserstoff, Niederalkyl, Niederalkenyl, Hydroxyniederalkyl, Niederalkoxyniederalkyl oder einen Phenylniederalkylrest darstellt, wobei in den genannten Gruppen Phenyl durch Niederalkyl, Niederalkoxy, Halogen, Trifluormethyl und/oder Nitro substituiert sein kann, und ihre Salze, Verfahren zu ihrer Herstellung, diese enthaltende pharmazeutische Präparate und ihre Verwendung.

Der gegebenenfalls wie angegeben substituierte Benzopyronrest R kann in beliebiger Stellung gebunden sein und ist beispielsweise ein gegebenenfalls substituierter, in 4-, 6- oder vor allem 3- oder 7-Stellung gebundener 2-Oxo-2H-1-benzopyranylrest oder in 3- oder in zweiter Linie 6- oder 7-Stellung gebundener 4-Oxo-4H-1benzopyranylrest.

An zwei benachbarte Kohlenstoffatome gebundenes Niederalkylen ist insbesondere in 5,6-, 6,7- oder 7,8-Stellung eines 4-Oxo-4H-1-benzopyranyl-3-restes oder in 3,4-Stellung eines 2-Oxo-2H-1-benzopyran-7-ylrestes gebundenes 3- oder 4-gliedriges Niederalkylen.

An zwei benachbarte Kohlenstoffatome gebundenes Niederalkylendioxy ist insbesondere in 5,6, 6,7- oder 7,8-Stellung gebundenes 3- oder 4-gliedriges Niederalkylendioxy.

Ein Benzoyloxyrest ist beispielsweise gegebenenfalls durch Niederalkyl, Niederalkoxy, Halogen, Trifluormethyl und/oder Nitro substituiertes Benzoyloxy.

Durch Niederalkyl oder Niederalkanoyl substituierte Aminogruppen sind z.B. N-Mono- oder N,N-Diniederalkylenamino oder Niederalkanoylamino.

Vor- und nachstehend haben die allgemeinen Begriffe folgende Bedeutung:

Niedere Reste enthalten beispielsweise bis zu 7, vor allem bis zu 4 Kohlenstoffatome.

Niederalkyl ist beispielsweise Methyl, Äthyl, Propyl, Isopropyl, n-Butyl, Isobutyl, sek.-Butyl oder tert.-Butyl, ferner n-Pentyl, n-Hexyl, Isohexyl oder n-Heptyl.

Niederalkylen ist beispielsweise 1,3-Propylen, 1,4-Butylen oder 1,5-Pentylen.

Phenylniederalkyl ist beispielsweise Benzyl, 1- oder 2-Phenyläthyl oder 3-Phenylpropyl.

Niederalkoxy ist beispielsweise Methoxy, Äthoxy, n-Propyloxy, Isopropyloxy oder n-Butyloxy.

Niederalkoxyniederalkyl ist beispielsweise 2-Methoxyäthyl oder 2-Äthoxyäthyl, während Hydroxyniederalkyl insbesondere 2-Hydroxyäthyl oder 3-Hydroxypropyl bedeutet.

Niederalkylendioxy ist beispielsweise 1,2-Äthylendioxy, 1,3-Propylendioxy oder Methylendioxy.

Niederalkanoyl ist beispielsweise Acetyl, Propionyl, Butyryl, Isobutyryl, Valeroyl oder Pivaloyl.

Niederalkanoyloxy ist beispielsweise Acetoxy, Propionyloxy, Butyryloxy, Isobutyryloxy, Valeroyloxy oder Pivaloyloxy.

Niederalkanoylamino ist beispielsweise Acetamino, Propionylamino oder Butyrylamino.

N-Mono- oder N,N-Diniederalkylamino ist beispielsweise N-Methyl-, N,N-Dimethyl- oder N,N-Diäthylamino.

Die Verbindungen der Formel I können in freier Form oder in Form ihrer Salze, insbesondere ihrer pharmazeutisch verwendbaren, nicht-toxischen Salzen vorliegen.

So können die Verbindungen der Formel I Salze mit Basen bilden, wie Salze von Metallen der Gruppe I und II des periodischen Systems der Elemente, z.B. Alkali- oder Erdalkalimetallsalze, insbesondere Natrium-, Kalium-, Magnesium oder Calciumsalze, Kupfer- oder Zinksalze, ferner Ammoniumsalze, sowie Salze mit organischen Basen, wie mit geeigneten Aminen, z.B. Äthylamin, Triäthylamin, Diäthylaminoäthanol, Äthylendiamin, Benzylamin, Prokain, Pyrrolidin, Piperidin, Morpholin, 1-Äthylpiperidin oder 2-Piperidinoäthanol.

Die Verbindungen der Formel I können in mehreren tautomeren Formen vorliegen. Die wichtigste dieser tautomeren Formen entspricht der Formel Ia

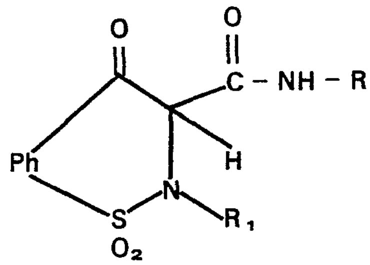

(Ia)

Verbindungen der vorliegenden Erfindung können ferner in Form von Gemischen von Isomeren, wie Racematen, oder von reinen Isomeren, z.B. Antipoden vorliegen.

Die erfindungsgemässen Azathianaphalinverbindungen, insbesondere die Verbindungen der Formel I, besitzen wertvolle pharmakologische, insbesondere antiinflammatorische Eigenschaften. Diese können z.B. anhand des Carrageneen-Pfotenödems der Ratte bei peroraler Applikation einer Dosis von jeweils etwa 30 bis 100 m/kg sowie anhand des Kaolin-Pfotenödems der Ratte bei peroraler Applikation einer Dosis von jeweils etwa 100 bis 300 mg/kg und anhand der Adjuvans-Arthritis der Ratte bei peroraler Applikation einer Dosis von jeweils etwa 30 bis 100 mg/kg dokumentiert werden. Die neuen 3,4-Dihydro-2H-1,2-benzothiazin-1,1-dioxide können daher als Antiinflammatorika bei entzündlichen Vorgängen verschiedener Äthiologie, vor allem des rheumatischen Formenkreises verwendet werden.

Aus der US-A-3591584 sowie «Journal of Medicinal Chemistry», Band 16(1), Januar 1973, Seite 44 bis 48 sind
2-Methyl-4-oxo-N-phenyl-3,4-dihydro-2H-1,2-benzothiazin-3-carboxamid-1,1-dioxid
sowie am N-Atom heterocyclische Substituenten aufweisende Derivate bekannt, wobei jedoch die heterocyclisch substituierten Derivate antiinflammatorisch geringer wirksam sind als die N-Phenylverbindung.

Die Erfindung betrifft vor allem Verbindungen der Formel I, worin Ph einen unsubstituierten oder durch Niederalkyl mit bis zu 4 Kohlenstoffatomen, wie Methyl, Niederalkoxy mit bis zu 4 Kohlenstoffatomen, wie Methoxy oder Äthoxy, Halogen bis Atomnummer 35, wie Chlor, Nitro oder Trifluormethyl substituierten 1,2-Phenylenrest bedeutet, R einen in 3-, 4-, 6- oder 7-Stellung gebundenen 2-Oxo-2H-1-benzopyranylrest oder in 3- oder in zweiter Linie 6- oder 7-Stellung gebundenen 4-Oxo-2H-1-benzopyranylrest darstellt, der unsubstituiert oder durch Niederalkyl mit bis zu 4 Kohlenstoffatomen, wie Methyl, Niederalkoxy mit bis zu 4 Kohlenstoffatomen, wie Methoxy, Halogen bis Atomnummer 35, wie Chlor, Niederalkanoyloxy mit bis zu 4 Kohlenstoffatomen, wie Acetoxy, Hydroxy, Amino, N-Mono- oder N,N-Diniederalkylamino mit bis zu 4 Kohlenstoffatomen im Alkylteil, wie Dimethylamino, Niederalkanyolamino mit bis zu 4 Kohlenstoffatomen, wie Acetylamino, und/oder an zwei benachbarten Kohlenstoffatomen durch Niederalkylen mit bis zu 4 Kohlenstoffatomen, wie 1,3-Propylen oder 1,4-Butylen, oder Niederalkylendioxy mit bis zu 4 Kohlenstoffatomen, wie Methylendioxy oder 1,2-Äthylendioxy, substituiert sein kann, und $R_1$ Wasserstoff oder Niederalkyl mit bis zu 4 Kohlenstoffatomen, wie Methyl oder Äthyl, bedeutet, und deren Salze mit Basen.

Die Erfindung betrifft ganz besonders Verbindungen der Formeln Ib und Ic

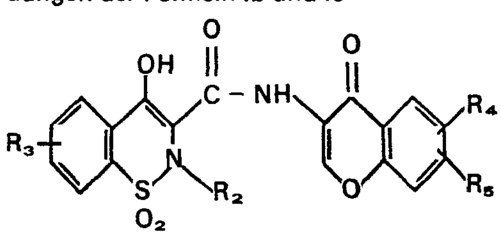 (Ib)

und

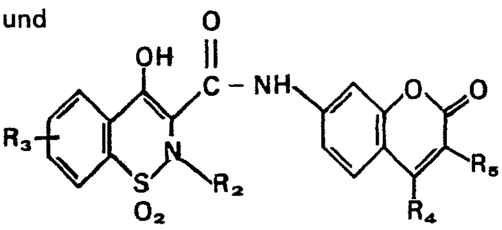 (Ic)

worin $R_2$ Wasserstoff oder vor allem Niederalkyl mit bis zu 4 Kohlenstoffatomen, z.B. Methyl oder Äthyl, bedeutet, $R_3$ Wasserstoff, Niederalkyl mit bis zu 4 Kohlenstoffatomen, z.B. Methyl, Niederalkoxy mit bis zu 4 Kohlenstoffatomen, z.B. Methoxy, Halogen bis Atomnummer 35, z.B. Chlor, Trifluormethyl oder Nitro bedeutet, und $R_4$ und $R_5$ unabhängig voneinander Wasserstoff, Niederalkyl mit bis zu 4 Kohlenstoffatomen, z.B. Methyl, oder Niederalkoxy mit bis zu 4 Kohlenstoffatomen, z.B. Methoxy, bedeuten oder gemeinsam einen an benachbarte Kohlenstoffatome gebundenen 3- oder 4-gliedrigen Niederalkylen- oder Niederalkylendioxyrest mit bis zu 4 Kohlenstoffatomen, z.B. 1,3-Propylen, 1,4-Butylen, Methylendioxy oder 1,2-Äthylendioxy, darstellen oder in Formel Ic $R_4$ Hydroxy und $R_5$ Wasserstoff oder Niederalkyl mit bis zu 4 Kohlenstoffatomen, wie Methyl, bedeutet, und ihre Salze mit Basen.

Die Erfindung betrifft in allererster Linie Verbindungen der Formel Ib, worin $R_2$ Niederalkyl mit bis zu 4 Kohlenstoffatomen, wie Methyl, bedeutet, $R_3$ Wasserstoff bedeutet, und $R_4$ und $R_5$ unabhängig voneinander Wasserstoff, Niederalkyl oder Niederalkoxy mit bis zu 4 Kohlenstoffatomen, wie Methyl oder Methoxy, oder gemeinsam, vorzugsweise in 6,7-Stellung gebundenes, 3- bis 4-gliedrieges Niederalkylen mit bis zu 4 Kohlenstoffatomen, wie 1,3-Propylen, bedeuten, und ihre Salze mit Basen.

Die Erfindung betrifft namentlich die in den Beispielen genannten Verbindungen der Formel I, insbesondere diejenigen der Formel Ib, und deren Salze mit Basen.

Die neuen Verbindungen der Formel I können nach an sich bekannten Methoden hergestellt werden, beispielsweise indem man eine Verbindung der Formel II

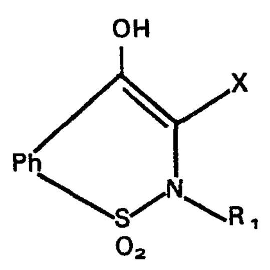 (II)

wobei X eine gegebenenfalls funktionell abgewandelte Carboxygruppe bedeutet, oder ein Salz davon, mit einer Verbindung der Formel $R-NH_2$ (III) oder mit einem reaktionsfähigen Derivat davon umsetzt, und, wenn erwünscht, eine verfahrensgemäss erhältliche Verbindung in eine andere Verbindung der Formel I überführt und/oder, wenn erwünscht, ein verfahrensgemäss erhältliches Salz in die freie Verbindung oder in ein anderes Salz oder ein verfahrensgemäss erhältliches Salz in die freie Verbindung umwandelt.

Funktionelle Carboxyderivate der Formel II sind vorzugsweise Ester, wie Niederalkylester, oder gegebenenfalls substituierte Phenylester, wie Phenylester oder 4-Nitro- oder 2,4-Dinitrophenylester, ferner oligomere, vorzugsweise dimere Lactone, Amide, wie primäre Amide, sekundäre Amide, z.B. Anilide, oder tertiäre Amide, z.B. 1-Imidazolide, N-Mono- oder N,N-Diniederalkylamide, Pyrrolidide, Piperidide oder Morpholide, und Anhydride, vorzugsweise gemischte Anhydride mit Mineralsäuren, wie Halogenwasserstoffsäuren, z.B. Chlorwasserstoffsäure, Anhydride mit phosphinigen oder phosphorigen Säuren, z.B. mit Diphenylphosphiniger Säure, mit Carbonsäuren, wie Niederalkancarbonsäuren, z.B. mit Ameisen- oder Essigsäure, oder mit Kohlensäurehalbestern oder sauren Estern von Sauerstoffsäuren des Phosphors, deren verbleibende Hydroxygruppe(n), z.B. mit einem Niederalkanol, verestert, z.B. mit einer Halogenwasserstoffsäure, anhydridisiert oder, z.B. mit dem Stickstoffatom in 2-Stellung einer Verbindung der Formel II, in der $R_1$ Wasserstoff ist, amidiert sind. Derartige funktionelle Carboxyderivate der Formel II sind z.B. Anhydride mit Kohlensäuremonophenyl- oder Kohlensäuremononiederalkylester bzw. Phosphorigsäure- oder Phosphorsäurediniederalkylester, oder cyclische Anhydride davon, wie Verbindungen der Formel IIa

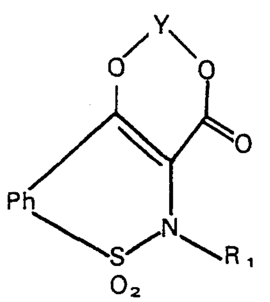

(IIa)

worin Y eine Carbonyl-, Thiocarbonyl- oder Sulfinylgruppe oder eine Gruppe der Formeln $>P-R_0$, $>P(=O)-R_0$ oder $>P(=O)\ (R_0)_2$, in der $R_0$ einen organischen Rest, wie Niederalkyl, z.B. Methyl, oder gegebenenfalls substituiertes Phenyl darstellt, bedeutet. Weitere funktionelle Carboxyderivate der Formel II sind deren Iminoäther (Iminoester) oder Säureadditionssalze derselben, wie offenkettige Iminoätherhydrohalogenide, z.B. Imidoniederalkylesterhydrochloride, oder cyclische Iminoäther, wie entsprechende 4,4- oder 5,5-Diniederalkyl-, z.B. 4,4- oder 5,5-Dimethyl-4,5-dihydrooxazole-(2) oder 4,4,6-Triniederalkyl-, z.B. 4,4,6-Trimethyl-5,6-dihydrooxazine-(2).

Reaktionsfähige Derivate von Aminen der Formel III sind insbesondere deren von Halbestern der Kohlensäure bzw. von Halogenameisensäure oder von organischen Sulfensäuren, wie Niederalkan- oder gegebenenfalls substituierten Benzolsulfensäuren abgeleitete Amide, z.B. Niederalkoxy-, wie Methoxy- oder Äthoxycarbonylderivate, Halogen-, wie Chlorcarbonylderivate, Niederalkan-, wie Methansulfenyl- oder Benzolsulfenylderivate derselben, oder entsprechende Carbonsäureamide, wie Niederalkanoyl-, z.B. Acetylderivate.

Die Umsetzung von Verbindungen der Formeln II und III bzw. von reaktionsfähigen Derivaten derselben erfolgt in der jeweils üblichen Weise.

Bei der Umsetzung von Säuren der Formel II mit Aminen der Formel III oder deren Salzen arbeitet man vorteilhaft in Gegenwart eines wasserbindenden Mittels, vorzugsweise von Phosphorpentoxid oder eines Esters der pyrophosphorigen Säure, z.B. von Tetraäthylpyrophosphit, oder unter destillativer, vorzugsweise azeotrop-destillativer, Entfernung des Reaktionswassers, erforderlichenfalls in einem inerten Lösungsmittel, wie Toluol, und/oder bei erhöhter Temperatur, z.B. bei etwa 50 bis 200°C.

Die Umsetzung von Estern oder Amiden oder von Anhydriden von Säuren der Formel II mit Aminen der Formel III oder deren Salzen wird vorteilhaft in einem gegenüber den Reaktionsteilnehmern inerten Lösungsmittel, z.B. in Toluol, Xylol, Tetrahydrofuran oder Dioxan, erforderlichenfalls in Gegenwart eines basischen Kondensationsmittels, wie einer tertiären organischen Stickstoffbase, wie Triäthylamin oder Pyridin, und/oder bei erniedrigter oder erhöhter Temperatur, z.B. im Temperaturbereich von etwa 0 bis etwa 150°C durchgeführt.

Die Umsetzung von Säuren der Formel II mit Acylderivaten von Aminen der Formel III erfolgt vorzugsweise unter Erhitzen, z.B. bei etwa 100 bis 250°C, erforderlichenfalls in einem gegenüber den Reaktionsteilnehmern inerten Lösungsmittel, wie Xylol, während man die Umsetzung mit von Aminen der Formel III abgeleiteten Sulfenylamiden vorzugsweise bei normaler Temperatur, z.B. bei etwa 0 bis 50°C, vorzugsweise in einem inerten Lösungsmittel, wie einem N,N-Diniederalkylamid, z.B. Dimethylformamid, oder N-Methylpyrrolidon, in Pyridin, in einem Äther, z.B. Diäthyläther, Dioxan oder Tetrahydrofuran, in Benzol, Toluol oder Xylol, durchführt.

Die Ausgangsstoffe der Formel II und III können in an sich bekannter Weise hergestellt werden.

So kann man Ester der Formel II beispielsweise erhalten, indem man einen Ester einer Säure der Formel IIb

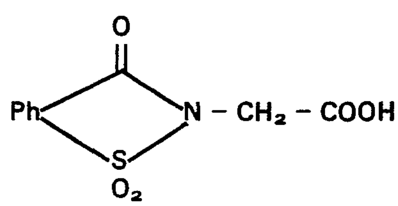

(IIb)

mit einem Äquivalent eines entsprechenden Alkalimetallalkoholates, z.B. Natriummethanolat, vorzugsweise in Dimethylsulfoxid oder Dimethylformamid, umsetzt. Aus den so erhältlichen Estern können sodann durch übliche Hydrolyse die freien Säuren, aus diesen deren Amide, z.B. ihre 1-Imidazolide durch Umsetzung mit bis-(1-Imidazolyl)harnstoff, durch Dehydratisierung deren Lactone bzw. Lactame oder durch Umsetzung mit einem Dihalogenid, Esterhalogenid oder Diester der Kohlensäure oder Thiokohlensäure, wie Phosgen, einem Halogen-, z.B. Chlorameisensäureniederalkylester oder -phenylester oder Thiophosgen, oder einem Diniederalkyl- oder Diphenylcarbonat bzw. -pyrocarbonat, mit einem Ester und/oder Halogenid einer phosphinigen Säure der Formel $(R_0)_2$ P−OH, wie einem Benzolphosphinigsäureniederalkyl, z.B. -äthylester, oder Benzolphosphinigsäurechlorid, einem Diester, Esterhalogenid oder Dihalogenid einer phosphonigen Säure der Formel $R_0-P(OH)_2$, z.B. mit Benzolphosphorigsäuredichlorid, einem Diester, Esterhalogenid oder Halogenid einer Phosphorsäure der Formel $R_0-P(=O)\ (OH)_2$, z.B. mit Benzolphosphonsäuredichlorid, oder mit Thionylchlorid, deren Anhydride hergestellt werden. Dabei werden cyclische Anhydride der Formel IIa vorteilhaft *in situ* gebildet und ohne Isolierung umgesetzt. Iminoäther (Iminoester) der Formel II können beispielsweise erhalten werden, indem man ein Nitril der Formel II in üblicher Weise, vorzugsweise säurekatalytisch, mit dem entsprechenden Alkohol, wie mit einem Niederalkanol, Phenol, Aminoniederalkanol oder Niederalkandiol, z.B. mit Methanol, Äthanol, Phenol, 4-Amino-2-methylpentan-2-ol oder 2-Methylpentan-2,4-diol, umsetzt. Aus den Nitrilen kann man ferner durch übliche Hydrolyse primäre Amide der Formel II erhalten. Nitrile der Formel II können in analoger Weise erhalten werden wie vorstehend für die entsprechenden Ester beschrieben, indem man von dem Nitril einer Säure der Formel IIb ausgeht. N-unsubstituierte Ester der Formel II und entsprechende Nitrile können in üblicher Weise, z.B. mit einem den Rest $R_1$ einführenden Mittel, N-substituiert werden.

Ausgangsstoffe der Formel III können beispielsweise hergestellt werden, indem man in einem entsprechenden Nitrooxobenzopyranderivat die Nitrogruppe in üblicher Weise, z.B. durch Behandeln mit durch Palladium auf Aktivkohle katalytisch aktiviertem Wasserstoff, z.B. in Dimethylformamid bei Normaldruck, zur Aminogruppe reduziert. Die vorstehend erwähnten Ausgangsstoffe sind ihrerseits bekannt oder können nach an sich bekannten Methoden hergestellt werden, 3-Nitro-4-oxo-4H-1-benzopyranderivate beispielsweise, indem man ein entsprechendes Methylsulfinylacetophenon der Formel

$HO-Ph-C(=O)-CH_2-S(=O)-CH_3$

in Gegenwart einer Base, z.B. von Kaliumcarbonat in Wasser, mit einem entsprechenden Aldehyd, z.B. Formaldehyd, kondensiert, aus dem so erhältlichen

3-Hydroxymethyl-3-methylsulfonyl-2,3-dihydro-4-oxo-4H-1-benzopyranderivat

thermisch, z.B. in siedendem Toluol, Methansulfinsäure abspaltet und das so erhältliche 3-Hydroxymethyl-4-oxo-4H-benzopyranderivat mit konzentrierter, z.B. 70%iger Salpetersäure, mässig, z.B. auf etwa 40°C, erwärmt. Eine direkte Bildungsweise für die erwähnten Nitroverbindungen besteht darin, dass man ein entsprechendes Nitroacetophenon in Gegenwart von Natriumformiat mit dem gemischten Anhydrid von Essig- und Ameisensäure, z.B. auf Siedetemperatur, erhitzt.

Aus Aminen der Formel III können in üblicher Weise, z.B. durch Umsetzung mit einem entsprechenden Halogenameisensäureester bzw. Carbonsäureanhydrid oder durch Umsetzung mit einem organischen Disulfid, z.B. einem Diniederalkyldisulfid, in Gegenwart einer organischen Phosphor-III-verbindung, z.B. Triphenylphosphin, deren reaktionsfähige Derivate erhalten werden. Von 3-Amino-4-oxo-4H-1-benzopyranen der Formel III abgeleitete Carbonsäureamide können ferner hergestellt werden, indem man ein entsprechendes 4-Oxo-2,3-dihydro-4H-1-benzopyran in üblicher Weise, z.B. durch Umsetzung mit Isoamylnitrit, in 3-Stellung oximiert und auf das erhaltene Oxim in Gegenwart des betreffenden Carbonsäureanydrides und des diesem entsprechenden Alkalimetallcarboxylates sowie von Palladium auf Bariumsulfat Wasser stoff einwirken lässt.

Die neuen Verbindungen können ferner hergestellt werden, indem man eine Verbindung der Formel IV

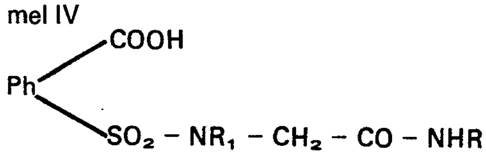

(IV)

oder ein funktionelles Carboxyderivat und/oder Salz davon zu einer Verbindung der Formel I cyclisiert und, wenn erwünscht, mindestens eine der genannten Zusatzmassnahmen durchführt.

Funktionelle Derivate von Verbindungen der Formel IV sind beispielsweise deren Ester, wie Niederalkylester oder Amide, wie unsubstituierte oder N-Mono- oder N,N-Diniederalkylamide oder Anilide, ferner von Verbindungen der Formel IV, in denen $R_1$ Wasserstoff ist, abgeleitete und mit diesen bzw. deren Estern und Amiden in einer dynamischen Gleichgewichtsbeziehung stehenden Lactame, z.B. solche der Formel

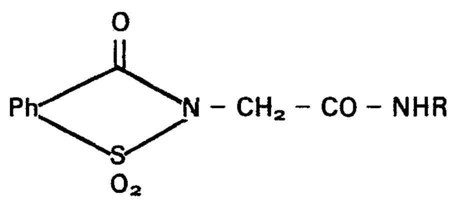

(IVa)

Salze von Verbindungen der Formel IV sind insbesondere deren Metall- oder Ammoniumsalze, insbesondere Alkalimetall-, z.B. Natrium- oder Kaliumsalze, oder Ammoniumsalze.

Die Cyclisierung von Verbindungen der Formel IV bzw. ihrer funktionellen Derivate und/oder Salze in Verbindungen der Formel I erfolgt in üblicher Weise, beispielsweise durch Behandlung mit einem basischen Kondensationsmittel. Als solche kommen insbesondere Metallbasen, wie Alkalimetallakoholate, insbesondere Natrium- oder Kaliumniederalkanolate, z.B. Natriummethanolat oder -äthanolat oder Kalium-tert.-butanolat, Alkalimetallamide, z.B. Natriumamid oder Lithiumdiisopropylamid oder Alkalimetall- oder Erdalkalimetallhydride, z.B. Natrium- oder Calciumhydrid, in Betracht. Die unter Ringerweiterung verlaufende Cyclisierung von Verbindungen der Formel IVa, für die mindestens zwei Äquivalente des basischen Kondensationsmittels erforderlich sind, kann ferner in Gegenwart organischer Stickstoffbasen, vorzugsweise tertiären Aminen, wie Triäthylamin, Pyridin oder Chinolin, oder von Alkalimetall- oder Ammoniumhydroxyden, z.B. von Kalium-, Natrium- oder Ammoniumhydroxyden, erfolgen. Die erfindungsgemässe Reaktion erfolgt gegebenfalls in Gegenwart eines Lösungs- oder Verdünnungsmittels, erforderlichenfalls unter Kühlen oder Erwärmen, z.B. im Temperaturbereich von etwa 0 bis etwa 150°C, in einem geschlossenen Gefäss und/oder unter Inertgas, wie Stickstoff.

Das Lösungsmittel wird dabei vor allem durch das zu verwendende Kondensationsmittel bestimmt. Bei der Behandlung mit einem Alkalimetallalkoholat verwendet man vorzugsweise den entsprechenden Alkohol, während man die Behandlung mit Alkalimetallamiden oder Alkalimetall- oder Erdalkalimetallhydriden vorzugsweise in einem N,N-Diniederalkylniederalkanoylamid, wie Dimethylformamid, oder einem Diniederalkylsulfoxid, wie Dimethylsulfoxid, die Behandlung mit einer organischen Stickstoffbase vorzugsweise in einem Überschuss derselben und die Behandlung mit einem Alkalimetall- oder Ammoniumhydroxid vorzugsweise in wässriger oder wasserhaltiger Lösung, z.B. in Wasser, Äthanol/Wasser oder Dimethylformamid/Wasser, durchführt.

Die Ausgangsstoffe der Formel IV und deren funktionelle Carboxyderivate sind bekannt oder können nach an sich bekannten Methoden hergestellt werden.

Verbindungen der Formel IVa können beispielsweise erhalten werden, indem man eine Verbindung der Formel IVb

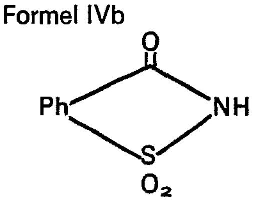

(IVb)

in üblicher Weise, z.B. in Analogie zur Bildung von Saccharin-Natrium, in ein Metallsalz überführt und dieses mit einer Verbindung der Formel $Hal-CH_2-CO-NHR$ (Hal = Chlor oder Brom) umsetzt.

Aus den z.B. so erhältlichen Verbindungen der Formel IVa können durch übliche Umsetzung mit einem Äquivalent eines Alkalimetallhydroxides, Alkalimetallalkoholates oder Amins Verbindungen der Formel IV bzw. deren Ester und Amide erhalten werden.

Die neuen Verbindungen können weiterhin hergestellt werden, indem man eine Verbindung der Formel V

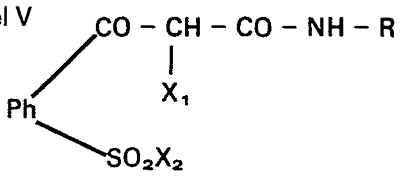

(V)

worin einer der Reste $X_1$ und $X_2$ eine Gruppe der Formel $-NHR_1$ und der andere eine gegebenenfalls verätherte oder reaktionsfähig veresterte Hydroxygruppe oder Aminogruppe $X_3$ bedeutet, intramolekular cyclisiert und, wenn erwünscht, eine oder mehrere der genannten Zusatzoperationen durchführt.

Verätherte Hydroxygruppen sind beispielsweise mit einem Niederalkanol veräthert. Reaktionsfähig veresterte Hydroxygruppen sind vorzugsweise Halogenatome, wie Chlor, Brom oder Jod. Amino ist beispielsweise solches der Formel $-NHR_1$.

Die intramolekulare Cyclisierung von Verbindungen der Formel V erfolgt in üblicher Weise, beispielsweise indem man die im Verlaufe der Reaktion abgespaltene Verbindung der Formel $HX_3$ in geeigneter Weise, z.B. auf physikalischem oder chemischem Wege, aus dem Reaktionsgemisch entfernt, beispielsweise durch Erhitzen in einem höher als die abgespaltene Verbindung $HX_3$ siedenden oder mit dieser ein azeotropes Gemisch bildenden Lösungs- oder Verdünnungsmittels, oder durch chemische Bindung als Salz, vorzugsweise Säureadditionssalz. So kann man Wasser, Alkohole und niedermolekulare Amine durch Destillation, entfernen. Halogenwasserstoffsäuren können ferner durch basische Kondensationsmittel, wie Alkalimetallhydroxide, -carbonate oder -hydrogencarbonate, z.B. Natriumhydroxid, Kaliumcarbonat oder Natriumbicarbonat, oder organische Stickstoffbasen, wie tertiäre Amine, z.B. Triäthylamine, Pyridin oder Chinolin, als Säureadditionssalze und Wasser durch ein wasserbindendes Mittel, wie Dicyclohexylcarbodiimid, chemisch gebunden werden. Die vorstehende Reaktion wird in üblicher Weise in An- oder Abwesenheit eines Lösungs- oder Verdünnungsmittels, erforderlichenfalls unter Erwärmen, z.B. im Temperaturbereich von etwa 20° bis 180°C, in einem geschlossenen Gefäss und/oder unter Inertgas, wie Stickstoff durchgeführt.

Die Ausgangsstoffe sind bekannt oder können in an sich bekannter Weise hergestellt werden, beispielsweise indem man eine Verbindung der Formel Va

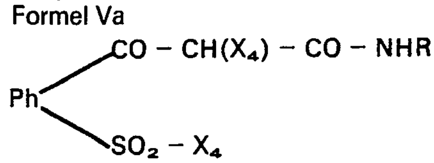

(Va)

worin mindestens einer der Reste $X_4$ Halogen, z.B. Chlor, und von Halogen verschiedener Rest $X_4$ eine Gruppe $-NHR_1$ bedeutet, mit einer Verbindung der Formel $R_1NH_2$ umsetzt.

Die Zwischenprodukte der Formel Va können z.B. erhalten werden, indem man einen Ester einer entsprechenden Verbindung der Formel $Ph(SO_2NHR_1)-COOH$, erhältlich durch Behandlung der entsprechenden Saccharinverbindung mit Kalilauge und übliche Veresterung an der Carboxygruppe, oder einen Ester einer entsprechenden Verbindung der Formel $Ph(SO_2Cl)-COOH$, erhältlich durch übliche Oxidation der entsprechenden o-Toluolsulfonsäure zur o-Sulfobenzoesäure, Veresterung derselben an der Carboxygruppe und Chlorierung der Sulfogruppe, z.B. mit Phosphoroxychlorid, in üblicher Weise mit einem Amid der Formel $CH_3-CO-NHR$ kondensiert, und das gebildete β-Ketosäureamid in α-Stellung halogeniert, z.B. mit Phosphorpentachlorid oder Sulfurylchlorid. Durch Umsetzung mit einer von Halogenwasserstoff verschiedenen Verbindung der Formel $HX_3$ können gewünschtenfalls andere Zwischenprodukte der Formel VIIa erhalten werden.

Die neuen Verbindungen können schliesslich hergestellt werden, indem man in einer Verbindung der Formel VI

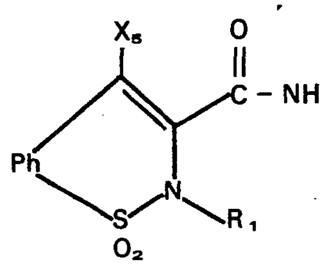
(VI)

worin $X_5$ einen in Hydroxy überführbaren Rest bedeutet, $X_5$ in Hydroxy überführt und gewünschtenfalls eine oder mehrere der genannten Zusatzoperationen durchführt.

In Hydroxy überführbare Reste $X_5$ sind beispielsweise verätherte oder veresterte Hydroxygruppen, gegebenenfalls substituierte Amino- oder Mercaptogruppen, Sulfinyl- oder Sulfonylgruppen oder Carboxy.

Verätherte Hydroxygruppen sind beispielsweise mit einem aliphatischen, araliphatischen oder aromatischen Alkohol verätherte Hydroxygruppen, wie Niederalkoxy oder gegebenenfalls substituierte Phenoxy- oder Benzyloxygruppen.

Veresterte Hydroxygruppen sind beispielsweise mit einer Halogenwasserstoffsäure oder mit einer Carbon- oder organischen Sulfonsäure veresterte Hydroxygruppen, wie Niederalkanoyloxy, gegebenenfalls substituierten Benzoyloxy, Niederalkan- bzw. Niederalkensulfonyloxy, z.B. Methan-, Äthan- oder Äthensulfonyloxy oder gegebenenfalls substituiertes Benzolsulfonyloxy; ferner Halogen, z.B. Chlor oder Brom.

Substituierte Aminogruppen sind beispielsweise durch gegebenenfalls substituierte Phenylgruppen, Niederalkyl, Niederalkylen bzw. Aza-, Oxa- oder Thiaalkylen mit bis zu 7 Kettengliedern substituierte Aminogruppen, wie gegebenenfalls substituiertes Anilino, N-Mono- oder N,N-Diniederalkylamino oder 5- bis 7-gliedriges 3-Aza-, 3-Oxa- oder 3-Thiaalkylenamino, z.B. Pyrrolidino, Piperidino oder Morpholino. Weitere substituierte Aminogruppen $X_5$ sind Diazoniumgruppierung, z.B. Diazoniumhalogenid- oder tetrafluoroboratgruppierungen, oder elektronegativ-monosubstituierte Aminogruppen, wie Hydroxyamino, gegebenenfalls phenyliertes Hydrazino, Acylamino, wie Niederalkanoylamino, z.B. Acetylamino oder Benzoylaminogruppen, Sulfonylamino, wie von aliphatischen oder aromatischen Sulfonsäuren abgeleitete Sulfonylaminogruppe, z.B. Methansulfonylamino, Benzolsulfonylamino, 4-Toluolsulfonylamino oder 4-Bromsulfonylamino.

Substituierte Mercaptogruppen sind beispielsweise durch aliphatische, cycloaliphatische oder aromatische Kohlenwasserstoffreste substituierte Mercaptogruppen, wie Niederalkylthio, z.B. Methylthio oder Äthylthio, Cycloalkylthiogruppen, z.B. Cyclohexylthio, oder Phenylthiogruppen.

Sulfonyl- bzw. Sulfinylgruppen sind beispielsweise Fluorsulfonyl bzw. Fluorsulfinyl oder aliphatische oder aromatische Sulfonyl- bzw. Sulfinylgruppen, wie Niederalkansulfonyl, z.B. Methan- oder Äthansulfonyl, Niederalkansulfinyl, z.B. Methan- oder Äthansulfinyl, Niederalkensulfonyl, z.B. Äthensulfonyl, oder Benzolsulfonyl- bzw. Benzolsulfinylgruppen.

In den genannten Gruppen $X_5$ können Phenylreste, beispielsweise durch Niederalkyl, wie Methyl, Niederalkoxy, wie Methoxy, Halogen, wie Chlor oder Brom, und/oder Nitro substituiert sein.

Die Überführung von Verbindungen der Formel VI in solche der Formel I erfolgt in üblicher Weise, beispielsweise durch Hydrolyse.

Die Hydrolyse kann in üblicher Weise durchgeführt werden, beispielsweise in Gegenwart eines, vorzugsweise basischen, Hydrolysemittels, z.B. von Natrium- oder Kaliumhydroxyd, bei normaler oder erforderlichenfalls erhöhter Temperatur, z.B. im Temperaturbereich von etwa 20 bis etwa 120°C, gegebenenfalls in Gegenwart eines Lösungsmittels und/oder weiterer Hilfsmittel. Als Lösungsmittel sind vor allem mit Wasser mischbare Lösungsmittel in Betracht zu ziehen, wie Niederalkanole, z.B. Methanol oder Äthanol, Äther, wie Dioxan, Diniederalkylcarbonsäureamide, wie Dimethylformamid, Sulfoxyde, wie Dimethylsulfoxid und Stickstoffbasen, wie Pyridin. Als weitere Hilfsmittel sind beispielsweise für die Hydrolyse von Carboxy zu Hydroxy einwertige Kupferverbindungen, z.B. Kupfer-I-chlorid, oder Kupfer-II-Verbindungen, z.B. Kupferoxid, zusammen mit metallischem Kupfer zu nennen.

Die Ausgangsstoffe sind bekannt oder können nach an sich bekannten Methoden hergestellt werden, beispielsweise, indem man eine Verbindung der Formel VIa

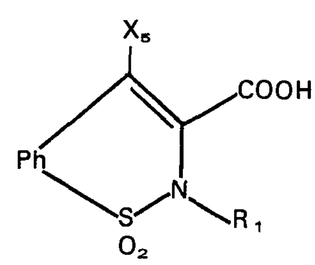
(VIa)

oder ein funktionelles Carboxyderivat davon, wie einen Ester, z.B. Niederalkylester, oder ein Anhydrid, wie das Chlorid, derselben in üblicher Weise mit einem Äquivalent eines Amins der Formel $R-NH_2$ (III) umsetzt.

Zwischenprodukte der Formel VI, worin $X_5$ Chlor ist, können ferner erhalten werden, indem man eine Säure der Formel VIb

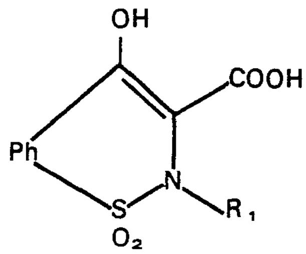

(VIb)

oder ein funktionelles Derivat, z.B. einen Niederlakylester, davon durch Umsetzung mit einem Chlorierungsmittel, z.B. mit Thionylchlorid, Phosphortrichlorid, Phosphoroxychlorid oder Phosphorpentachlorid, an der 4-Hydroxygruppe mit Chlorwasserstoffsäure verestert und gegebenenfalls an der Carboxygruppe mit Chlorwasserstoffsäure anhydridisiert.

Aus den genannten Chlorverbindungen können in analoger Weise, beispielsweise durch Umsetzung mit Thioharnstoff oder einem Alkalimetallalkanthiocarbonsäuresalz, z.B. mit Natriumthiolacetat, und nachfolgende Hydrolyse oder durch Umsetzung mit einem Alkalimetall-, wie Natriumsalz eines Mercaptans, die entsprechenden Verbindungen der Formel VI hergestellt werden, in denen $X_5$ eine gegebenenfalls substituierte Mercaptogruppe ist. Aus den Mercaptanen können sodann durch Einwirkung eines üblichen S-Oxydationsmittel, z.B. von 3-Chlorperbenzoesäure, die diesen entsprechenden Sulfonyl- bzw. Sulfinylverbindungen erhalten werden. Analog kann man durch Umsetzung mit dem entsprechenden Alkohol bzw. der entsprechenden Carbonsäure oder einem Alkalimetallsalz, z.B. dem Natriumsalz, davon oder mit Ammoniak bzw. einem entsprechenden Amin Verbindungen der Formel VI herstellen, in denen $X_5$ veräthertes oder verestertes Hydroxy oder gegebenenfalls anders als durch elektronegative Substituenten substituiertes Amino ist. Primäre Amino $X_5$ kann sodann durch Umsetzung mit einem elektronegativen Substituenten einführenden Mittel, z.B. mit einem Carbonsäureanhydrid oder -halogenid oder einem Sulfonsäurehalogenid oder durch übliche Diazotierung, elektronegativ substituiert werden. Die vorstehenden gegenseitigen Umwandlungen von Gruppen $X_5$ können dabei an Verbindungen der Formeln VI oder VIa bzw. im letzteren Fall deren Estern, durchgeführt werden.

Ausgangsstoffe der Formel VI worin $X_5$ veräthertes Hydroxy ist, können ferner erhalten werden, indem man eine Säure der oben angegebenen Formel VIb bzw. einen Ester davon, an der alkoholischen und an einer gegebenenfalls vorhandenen carboxylischen Hydroxygruppe veräthert, z.B. mit einem Niederalkylhalogenid oder Diniederalkylsulfat in Gegenwart von Kaliumcarbonat in Aceton oder Amylalkohol, und den so erhältlichen Ester in üblicher Weise, erforderlichenfalls nach Hydrolyse zur Säure und/oder Chlorierung derselben, z.B. mit Thionylchlorid, mit einem Amin der Formel III umsetzt.

Ausgangsstoffe der Formel VI worin $X_5$ eine disubstituierte Aminogruppe bedeutet, können ferner hergestellt werden, indem man eine Verbindung der Formel VIc

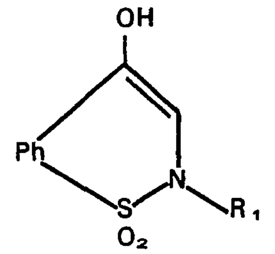

(VIc)

zunächst durch Umsetzung mit dem entsprechenden Amin in das Enamin überführt, dieses in Gegenwart von Triäthylamin mit Phosgen umsetzt und das so erhältliche Enaminsäurechlorid anschliessend in üblicher Weise mit einem Amin der Formel $R-NH_2$ (III) zur Reaktion bringt.

Die Verbindungen der Formel I können ferner hergestellt werden, indem man in einer Verbindung der Formel VII

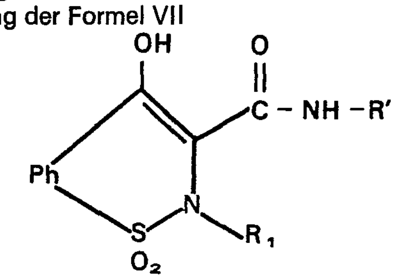

(VII)

worin R' einen zu dem gegebenenfalls substituierten Benzopyronrest R cyclisierbaren Rest bedeutet, oder in einem Salz davon R' zu dem Rest R ringschliesst und gewünschtenfalls eine so erhältliche Verbindung in eine andere Verbindung der Formel I und/oder eine erhaltene freie Verbindung in ein Salz oder ein erhaltenes Salz in die freie Verbindung oder in ein anderes Salz überführt.

Zu Benzopyronresten R cyclisierbare Reste R' sind beispielsweise zu Resten R der Formel VIIa

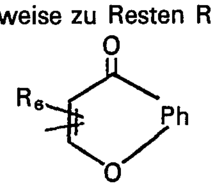

(VIIa)

cyclisierbare Reste der Formeln VIIa1, VIIa2, VIIa3 bzw. VIIa4

$- CH_2 - C(=O) - Ph - O - C(=O) - R_6$ (VIIa1)

$- CH(COR_6) - C(=O) - Ph - R_8$ (VIIa2)

$- C(=O) - CH(R_6) - C(=O) - Ph - R_8$ (VIIa3) bzw.

$- C(R_9)=C(R_6) - O - Ph - H$ (VIIa4)

zu Resten R der Formel VIIb

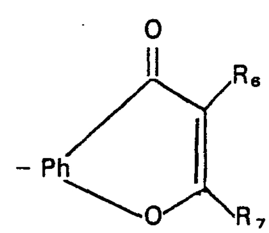

(VIIb)

cyclisierbare Reste der Formeln VIIb1, VIIb2 bzw. VIIb3

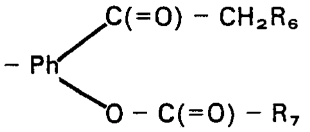

(VIIb1)

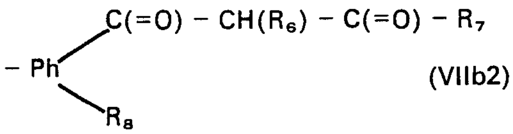

(VIIb2) bzw.

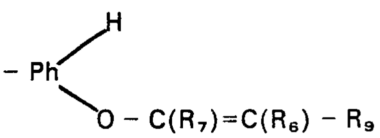

(VIIb3)

zu Resten R der Formel VIIc

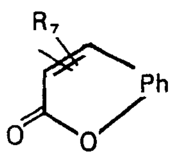

(VIIc)

cyclisierbare Reste der Formeln VIIc1, VIIc2, VIIc3 bzw. VIIc4

$- C(R_9)=C(R_7) - Ph - R_8$ (VIIc1)

$- C(PhR_8)=C(R_7) - R_9$ (VIIc2)

$- CH_2 - C(=O) - O - Ph - R_9$ (VIIc3) bzw.

$- CH(R_9) - C(=O) - Ph - R_8$ (VIIc4)

oder zu Resten der Formel VIId

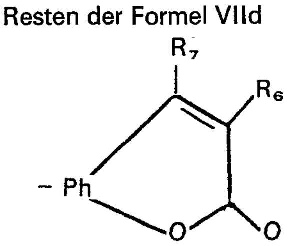

(VIId)

$- Ph(R_8) - C(R_7)=C(R_6) - R_9$ (VIId1)

$- Ph(R_9) - O - C(=O) - CH_2R_6$ (VIId2)

$- Ph(R_8) - C(=O) - CH(R_6) - R_9$ (VIId3) bzw.

$- Ph(O - R_9) - C(=O) - CH_2 R_8$ (VIId4),

worin Ph die eingangs angegebene Bedeutung hat, $R_6$ Wasserstoff einen aliphatischen Kohlenwasserstoffrest oder einen gegebenenfalls substituierten Phenyl- oder Pyridylrest bedeutet, $R_7$ eine der für $R_6$ angegebenen Bedeutungen hat, Hydroxy bedeutet oder gemeinsam mit $R_6$ einen zweiwertigen aliphatischen Kohlenwasserstoffrest darstellt, $R_8$ gegebenenfalls veräthertes oder mit einer Carbonsäure verestertes Hydroxy bedeutet und $R_9$ gegebenenfalls verestertes oder anhydrisiertes Carboxy darstellt.

Aliphatische Kohlenwasserstoffreste $R_6$ bzw. $R_7$ sind insbesondere Niederalkylreste. Als Substituenten von Phenylresten $R_6$ bzw. $R_7$ kommen beispielsweise Niederalkyl, Niederalkoxy, Halogen und/oder Nitro, als Substituenten von Pyridylresten $R_6$ bzw. $R_7$ insbesondere Niederalkyl in Betracht. Zweiwertige aliphatische Reste $R_6 + R_7$ sind insbesondere 3- bis 5-gliedrige Alkylenreste, wie 1,3-Propylen und 1,4-Butylen. Veräthertes Hydroxy ist beispielsweise Niederalkoxy, wie Methoxy, oder gegebenenfalls wie für Phenyl $R_6$ bzw. $R_7$ angegebenen substituiertes Phenoxy. Mit einer Carbonsäure verestertes Hydroxy ist beispielsweise Niederalkanoyloxy, wie Formyloxy oder Acetoxy, oder wie für Phenyl $R_6$ bzw. $R_7$ angegeben substituiertes Benzoyloxy. Verestertes Carboxy ist beispielsweise Niederalkoxycarbonyl, wie Methoxy- oder Äthoxycarbonyl, oder gegebenenfalls wie für Phenyl $R_6$ bzw. $R_7$ angegeben substituiertes Phenoxycarbonyl. Anhydridisiertes Carboxy ist beispielsweise Niederalkanoyloxycarbonyl, wie Formyl- oder Acetoxycarbonyl, oder Halogen, wie Chlorcarbonyl.

Die Cyclisierung von Verbindungen der Formel VII erfolgt in üblicher, beispielsweise in der aus der chemischen Literatur für analoge Reaktionen bekannten Weise, vorzugsweise in Gegenwart eines basischen oder sauren Kondensationsmittels und in einem gegenüber den Reaktionsteilnehmern inerten Lösungsmittel, erforderlichenfalls unter Entfernung von bei der Kondensation gebildeten flüchtigen Verbindungen durch, beispielsweise azeotrope, Destillation, unter Kühlen oder Erwärmen, z.B. im Temperaturbereich von etwa 0 bis etwa 200°C, unter Inertgas, wie Stickstoff, und/oder in geschlossenem Gefäss. Basische Kondensationsmittel sind beispielsweise Alkalimetallhydroxide, -alkoholate, wie -niederalkanolate, -carbonate oder -carboxylate, wie -niederalkansäurecarboxylate, z.B. Natrium- oder Kaliumhydroxid, Natrium- oder Kaliummethanolat oder -äthanolat, Kaliumcarbonat oder Natrium- oder Kaliumacetat. Saure Kondensationsmittel sind beispielsweise Protonensäuren oder ihre Anhydride, wie Schwefelsäure, Phosphorsäure, Halogen-, z.B. Chlor-, Brom- oder Jodwasserstoffsäure, Phosphorpentoxid oder Acetylchlorid bzw. Acetanhydrid, ferner Lewissäuren, wie Aluminiumtrichlorid. Geeignete Lösungsmittel sind beispielsweise Kohlenwasserstoffe, wie Benzol, Toluol oder Xylol, Alkohole wie Niederalkanole oder Niederalkandiole, z.B. Äthanol, Methanol oder Äthylenglykol, Diniedralkylketone, wie Aceton, aliphatische oder cycloaliphatische Äther, wie Tetrahydrofuran oder Dibutyläther, Carbonsäuren, wie Essigsäure, bzw. deren Anhydride, wie Acetanhydrid, oder tertiäre Niederalkansäureamide, wie Dimethylformamid oder N-Methylpyrrolidon, oder Gemische, z.B. wässrige Gemische derselben. Zur Cyclisierung von Verbindungen der Formel VII, worin R' einen Rest der Formel VIIa2, VIIa3, VIIa4, VIIb2, VIIb3, VIIc1, VIIc2, VIIc4,

VIId1 oder VIId3 bedeutet, verwendet man vorzugsweise saure Kondensationsmittel, wie starke, z.B. mindestens 20%ige, wässrige Jodwasserstoffsäure oder Bromwasserstoff in Essigsäure, im Falle von Resten R' der Formeln VIIc4 oder VIId3, z.B. konzentrierte, d.h. mindestens 50%ige, Schwefelsäure und im Falle von Resten R' der Formel VIIa4 oder VIIb3 z.B. ebenfalls Schwefelsäure aber auch Säureanhydride, z.B. Phosphorpentoxid oder Acetylchlorid. Ausgehend von Verbindungen der Formel VII, in denen R' einen Rest der Formeln VIIa1, VIIb1, VIIc3, VIIc4, VIId3 oder VIId4 bedeutet, arbeitet man vorzugweise in Gegenwart eines basischen Kondensationsmittels, im Falle von Resten R' der Formel VIIa1 oder VIIb1, z.B. von Kaliumcarbonat in Aceton oder eines Natrium- oder Kaliumsalzes einer organischen Carbonsäure im Gegenwert des flüchtigen Anhydrides derselben, oder im Falle von Resten R' der Formeln VIIc3, VIIc4, VIId3 oder VIId4 von Natrium oder eines Alkalimetall-, vorzugsweise Natriumalkoholates in dem betreffenden Alkohol.

Die Ausgangsstoffe der Formel VII können, soweit sie neu sind, nach an sich bekannten Methoden hergestellt werden, beispielsweise, indem man einen Ester, wie Niederalkylester einer Säure der Formel

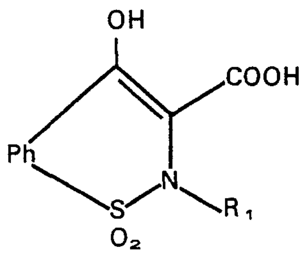

(VIII)

mit einem Amin der Formel $R'-NH_2$ umsetzt, vorzugsweise, wie für die Umsetzung von Estern der Formel II mit Aminen der Formel III angegeben.

Verbindungen der Formel VII, in denen R' einen Rest der Formel VIIa2 darstellt, können ferner erhalten werden, indem man z.B. Verbindungen der Formel IX

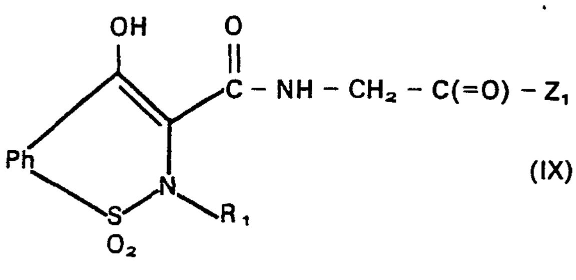

(IX)

in Gegenwart des gemischten Anhydrides von Ameisensäure und Essigsäure mit einem Salz, z.B. dem Natriumsalz einer Säure der Formel $Z_2-COOH$ (X) wobei einer der Reste $Z_1$ und $Z_2$ eine Gruppe $R_6$ und der andere eine Gruppe $-PhR_8$ bedeutet. In analoger Weise kann man Verbindungen der Formel VII in denen R' einen Rest der Formel VIIb2 darstellt, ferner erhalten, indem man eine Säure der Formel XI

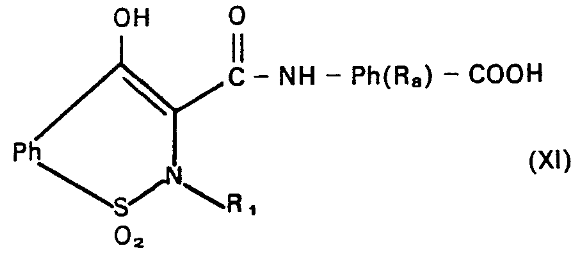

(XI)

oder einen Ester, z.B. Niederalkylester, oder Salz, z.B. Natriumsalz davon mit einer Verbindung der Formel $R_6CH_2(=O)-R_7$ (XII) bzw. einem Ester und/oder Salz, z.B. dem Natriumsalz, davon umsetzt, beispielsweise wie vorstehend angegeben oder in Gegenwart von Natrium in Xylol oder von Natriummethanolat in Methanol. Verbindungen der Formel VII, in denen R' einen Rest der Formel VIIb3 bedeutet, kann man ferner erhalten, indem man das von einer Verbindung der Formel XIII

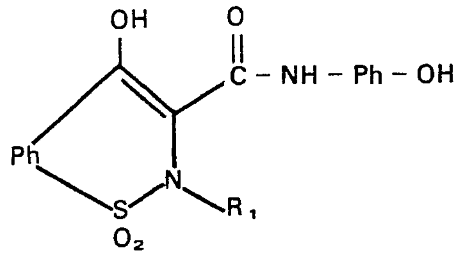

(XIII)

abgeleitete Alkalimetall-, wie Natriumphenolat mit einer Verbindung der Formel XIV

$Hal-C(R_7) = C(R_6)-R_9$, (XIV)

worin Hal = Halogen, wie Chlor oder Brom, darstellt oder vorzugsweise einem Salz oder Ester, wie Niederalkylester, davon umsetzt. Als Rest R' eine Gruppe der Formel VIId1 aufweisende Verbindungen der Formel VII kann man ferner durch übliche Umsetzung einer Verbindung der Formel XVI

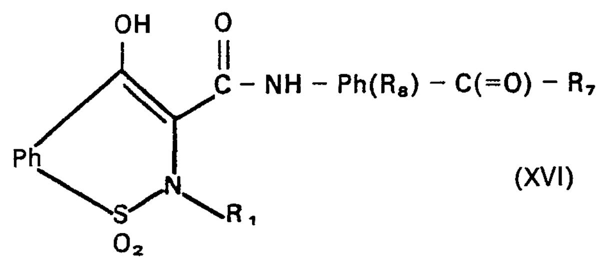

(XVI)

mit einer Verbindung der Formel

$R_6CH_2R_9$, (XVII) erhalten.

Ausgangsstoffe der Formel VII, worin R' einen Rest der Formeln VIIa1, VIIb1, VIIc3, VIIc4, VIId3 oder VIId4 darstellt, werden vorteilhaft unter den Reaktionsbedingungen *in situ* hergestellt und ohne Isolierung cyclisiert. So kann man beispielsweise eine Verbindung der Formel XVIII

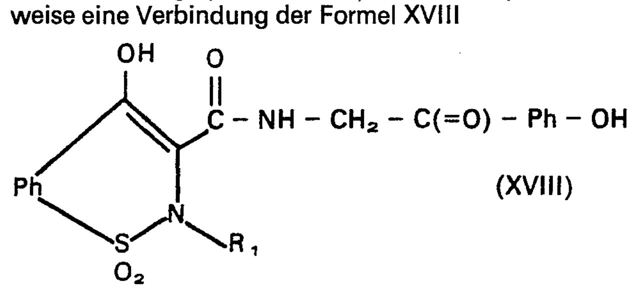

(XVIII)

mit einem Anhydrid einer Säure der Formel $R_6$ COOH (XIX) umsetzen, vorteilhaft in Gegenwart eines Alkalimetall- z.B. des Natriumsalzes, derselben, wobei intermediär eine Verbindung der Formel VII gebildet wird, in der R' einen Rest der Formel VIIa1 darstellt. Analog kann man eine Verbindung der Formel XX

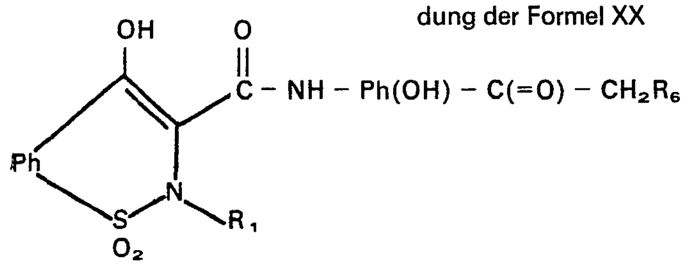

(XX)

unter intermediärer Bildung einer Verbindung der Formel VII, worin R' eine Gruppe der Formel VIIb1 bedeutet, mit einem Anhydrid einer Verbindung der Formel $R_7$ COOH, (XXI) worin $R_7$ von Hydroxy verschieden ist, umsetzen. Ferner kann man in Gegenwart starker Basen, z.B. von Natrium, oder von Alkalimetall-, z.B. Natriumalkoholaten Verbindungen der Formeln XXII

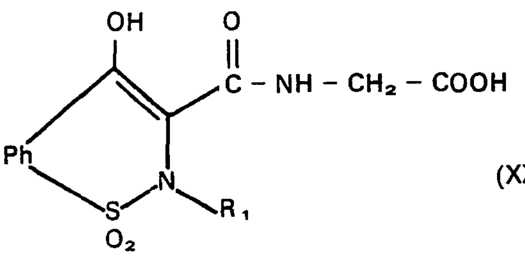

(XXII)

bzw. einen Ester oder ein Salz davon und HO–Ph $R_9$ (XXIII) oder ein Salz davon unter intermediärer Bildung eines Restes R' der Formel VIIc3, oder Verbindungen der Formeln XXIV

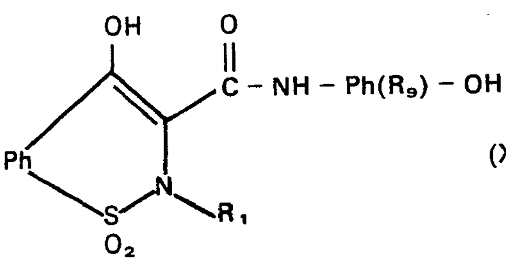

(XXIV)

bzw. einen Ester und/oder ein Salz davon und $R_6CH_2COOH$, (XXV) bzw. einen Ester oder ein Salz davon unter intermediärer Bildung eines Restes R' der Formel VIId2, oder Verbindungen der Formeln XXVI

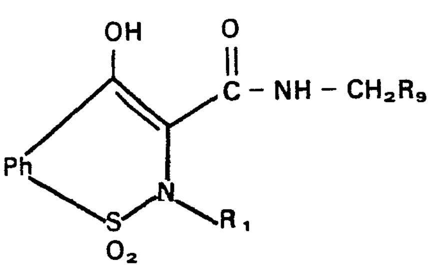

(XXVI)

bzw. einen Esters und/oder ein Salz davon und $R_8$–Ph–COOH (XXVII) bzw. einen Esters oder ein Salz davon unter intermediärer Bildung eines Restes R' der Formel VIIc4, oder Verbindungen der Formel XXVIII

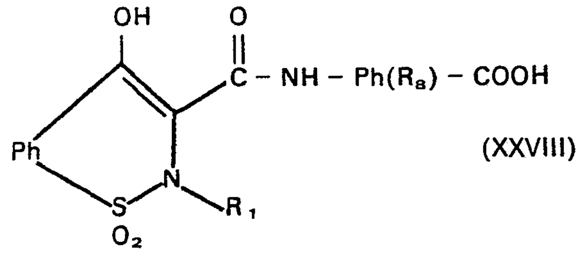

(XXVIII)

bzw. einen Ester und/oder Salz davon und der Formel $R_6CH_2R_9$, (XXIX) oder einen Ester oder ein Salz davon unter intermediärer Bildung eines Restes R' der Formel VIId3 kondensieren. Ferner kann man in vorteilhafter Weise eine Verbindung der Formel XXX

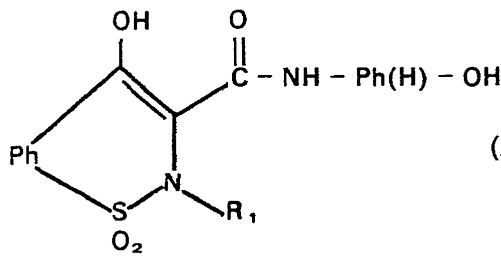

(XXXII)

mit einer Verbindung der Formel
$R_7$–C(=O)–CH($R_6$)$R_9$ (XXXI)
in Gegenwart eines sauren Mittels, wie einer Protonensäure, z.B. von Schwefelsäure, oder eines Säureanhydrides, z.B. von Phosphorpentoxid, umsetzen. Bei dieser Umsetzung, die unter Bildung einer nicht genau bekannten Zwischenstufe als erste isolierbare Reaktionsprodukt direkt die entsprechenden Benzopyronderivate der Formel I liefert, wird das Verhältnis der resultierenden 2H-1-Benzopyron- und 4H-1-Benzopyronverbindung von dem spezifischen Ausgangsstoffen und den Reaktionsbindungen bestimmt. Im allgemeinen werden jedoch bei Verwendung von Phosphorpentoxid als Kondensationsmittel überwiegend 4H-1-Benzopyronderivate, bei Verwendung mehr oder weniger konzentrierter Schwefelsäure hingegen überwiegend 2H-1-Benzopyronderivate erhalten.

Eine verfahrensgemäss erhältliche Verbindung der Formel I kann in üblicher Weise in eine andere Verbindung der Formel I umgewandelt werden.

Beispielsweise kann man in eine erfindungsgemäss erhältliche Verbindung der Formel I, worin $R_1$ Wasserstoff bedeutet, einen gegebenenfalls substituierten Kohlenwasserstoffrest aliphatischen Charakters einführen, beispielsweise durch

Umsetzung mit einem diesen einführenden Mittel, wie einem reaktionsfähigen Ester, z.B. Mineralsäure- oder Sulfonsäureester, des betreffenden Alkohols, oder einem von diesem abgeleiteten Epoxid. Mineralsäureester sind dabei vor allem Halogen-, wie Chlor-, Brom- oder Jodwasserstoffsäureester, sowie Schwefelsäureester und Sulfonsäureester vor allem Ester mit aromatischen oder aliphatischen Sulfonsäuren, wie Methan-, Äthan-, Äthen-, Benzol, p-Brombenzol-oder p-Toluolsulfonsäureester. Epoxide sind beispielsweise von Niederalkyl- oder Phenylniederalkylresten $R_1$ mit mindestens zwei Kohlenstoffatomen im Alkylteil abgeleitete Epoxide. In analoger Weise kann man auch primäre Amino und Hydroxy als Substituenten von R durch aliphatische Kohlenwasserstoffreste oder Hydroxy an 1,2-Phenylenreste Ph durch Niederalkyl veräthern.

In analoger Weise kann man weiterhin primäres Amino und/oder Hydroxy im Rest R acylieren, beispielsweise durch Umsetzung mit einem Carbonsäureanhydrid, wie einem Niederalkansäure- oder gegebenenfalls substituierten Benzoesäureanhydrid oder einem entsprechenden Halogenid, wie Chlorid.

Die vorstehenden Umsetzungen werden in üblicher Weise durchgeführt, erforderlichenfalls in Gegenwart eines basischen Kondensationsmittels, wie einer Metallbase, z.B. eines Alkalimetall- oder Erdalkalimetallhydroxydes, -carbonates, -hydrides oder einer Alkalimetallkohlenwasserstoffverbindung, einer Stickstoffbase, z.B. eines tertiären Amins, erforderlichenfalls in einem inerten Lösungsmittel und/oder unter Kühlen oder Erwärmen, z.B. im Temperaturbereich von etwa −10 bis etwa +105°C. Metallbasen sind insbesondere Natrium- und Kaliumhydroxid bzw. -carbonat, Natriummethanolat, Natriumhydrid, Diisopropylaminlithium und Phenyl- sowie Butyllithium. Stickstoffbasen sind insbesondere Pyridin, Triäthylamin oder Chinolin.

Umgekehrt kann man in erfindungsgemäss erhältlichen Verbindungen in üblicher Weise Acylamino zu Amino und/oder Acyloxy und/oder veräthertes Hydroxy zu Hydroxy hydrolysieren, beispielsweise in Gegenwart eines sauren oder basischen Hydrolysemittels, erforderlichenfalls unter gelindem Erwärmen, z.B. im Temperaturbereich von etwa 10 bis etwa 100°C, zu den entsprechenden Amino- bzw. Hydroxyverbindungen der Formel I hydrolysiert. Saure Hydrolysemittel sind insbesondere Mineralsäuren, wie Salz- oder Schwefelsäure, oder organische Carbon- oder Sulfonsäuren, wie Essigsäure oder p-Toluolsulfonsäure. Basische Hydrolysemittel sind insbesondere Alkalien, wie Natrium- oder Kaliumhydroxyd- oder -carbonat. Für die Hydrolyse von verätherten Hydroxygruppen verwendet man ferner Bromwasserstoffsäure in Essigsäure, Chlorwasserstoffsäure in Pyridin oder Jodwasserstoffsäure als mineralsaures Hydrolysemittel.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Je nach Verfahrensbedingungen und Ausgangsstoffen erhält man gegebenenfalls salzbildende Endstoffe in freier Form oder in Form ihrer Salze, die sich in üblicher Weise ineinander oder in andere Salze umwandeln lassen. So kann man die Verbindungen der Formel I in Form ihrer Salze mit Basen erhalten; diese können in üblicher Weise, z.B. durch Umsetzen der freien Verbindung mit einem entsprechenden basischen Mittel, wie einem Hydroxyd eines Metalls der Gruppen I und II des Periodischen Systems der Elemente, z.B. Alkali- oder Erdalkalimetallhydroxid, -carbonat, -hydrogencarbonat, -amid oder -hydrid oder einem geeigneten Alkalimetallniederalkanolat, mit Kupfer- oder Zinkoxid, oder mit Ammoniak oder einem Amin in ein Salz, vor allem in ein pharmazeutisch verwendbares Salz übergeführt werden. Aus entsprechenden Salzen lassen sich freie Verbindungen der Formel I in üblicher Weise, z.B. durch Umsetzen mit sauren Mitteln freisetzen.

Diese und andere Salze können zur Reinigung der neuen Verbindungen verwendet werden, z.B. indem man die freien Verbindungen in ihre Salze überführt, diese isoliert und wieder in die freien Verbindungen überführt. Infolge der engen Beziehung zwischen den neuen Verbindungen in freier Form und in Form ihrer Salze sind im vorausgegangenen und nachfolgend unter den freien Verbindungen sinn- und zweckmässig gegebenenfalls auch die entsprechenden Salze zu verstehen.

Erhaltene Isomerengemisch können auf Grund der physikalisch-chemischen Unterschiede der Bestandteile in bekannter Weise in die beiden Stereoisomeren, reinen Isomere aufgetrennt werden, beispielsweise durch Chromatographie und/oder fraktionierte Kristallisation. Vorteilhafterweise isoliert man das wirksamere der Isomeren.

Die Erfindung betrifft auch diejenigen Ausführungsformen des Verfahrens, nach dem man von einer auf irgendeiner Stufe des Verfahrens als Zwischenprodukt erhältlichen Verbindungen ausgeht, und die fehlenden Verfahrensschritte durchführt, oder bei denen man einen Ausgangsstoff unter den Reaktionbedingungen bildet, oder bei denen eine Reaktionskomponente gegebenenfalls in Form ihrer Derivate, wie ihrer Salze und/oder in Form von Isomerengemischen oder einen Isomeren einsetzt.

Zweckmässig verwendet man für die Durchführung der erfindungsgemässen Reaktionen solche Ausgangsstoffe, die zu den Eingangs besonders erwähnten Gruppen von Endstoffen und vor allem zu den speziell beschriebenen oder hervorgehobenen Endstoffen führen.

Bei den erfindungsgemässen pharmazeutischen Präparaten, welche Verbindungen der Formel I oder pharmazeutisch verwendbare Salze von solchen Verbindungen mit salzbildenden Gruppen enthalten, handelt es sich um solche zur enteralen, wie oralen oder rektalen, sowie parenteralen Verabreichung an Warmblüter, welche den pharmakologischen Wirkstoff allein oder zusammen mit einem pharmazeutisch anwendbaren Trägermaterial enthalten.

Die neuen pharmazeutischen Präparate enthalten von etwa 10 bis etwa 95%, vorzugsweise von etwa 20 bis etwa 90% des Wirkstoffes. Erfindungsgemässe pharmazeutische Präparate in Dosiseinheitsform, wie Dragées, Tabletten, Kapseln, Suppositorien oder Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister z.B. Mais-, Weizen-, Reis- oder Kartoffelstärke, Kleister, Gelatine, Tragakanth, Methylcellulose, Hydroxypropylmethylcellulose, Natriumcarboxymethylcellulose, und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessreglier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten. Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragéeüberzügen können Farbstoffe oder Pigmente, z.B. zur Identifizierung oder zur Kennzeichnung verschiedener Wirkstoffe beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die erfindungsgemässen Verbindungen der Formel I oder deren pharmazeutisch verwendbare Salze können als pharmakologisch wirksame Stoffe, insbesondere als Antiphlogistika, sowie als Analgetika, vorzugsweise in Form von pharmazeutischen Präparaten verwendet werden. Die Dosierung des Wirkstoffes hängt vom Warmblüterspezies, dem Körpergewicht und Alter und vom individuellen Zustand, sowie von der Applikationsweise ab. Durchschnittlich wird einem Warmblüter von etwa 70 kg Körpergewicht eine Tagesdosis von etwa 50 bis etwa 200 mg, vorzugsweise von etwa 75 bis etwa 150 mg Wirkstoff verabreicht.

Die nachfolgenden Beispiele illustrieren die obenbeschriebene Erfindung; sie sollen jedoch diese in ihrem Umfang in keiner Weise einschränken. Temperaturen werden in Celsiusgraden angegeben.

*Beispiel 1:*

In einem 500 ml fassenden Rundkolben, der mit einem Destillierkopf und einem Kondensator verbunden war, wurden
3,5 g (0,013 mol) 2-Methyl-3,4-dihydro-4-oxo-2H-1,2-benzothiazin-3-carbonsäuremethylester-1,1-dioxid, 2,1 g (0,013 mol) 3-Amino-4-oxo-4H-1-benzopyran
und 100 ml trockenes Xylol eingesetzt. Dann wurde in der erhaltenen Suspension 5 Minuten lang Stickstoffgas eingeblasen. Anschliessend wurde das Reaktionsgemisch einer 15 Stunden währenden langsamen Destillation unterworfen, wobei bereits nach 10 Minuten Erhitzen vollständige Lösung eintrat. Das Lösungsmittel wurde alle zwei Stunden ersetzt. Anschliessend wurde auf 50°C abgekühlt, der sich gebildete Niederschlag abfiltriert und mit Äthanol gewaschen. Nach dem Trocknen erhält man das
2-Methyl-3,4-dihydro-4-oxo-N-(4-oxo-4H-1-benzopyran-3-yl)-2H-1,2-benzothiazin-3-carboxamid-1,1-dioxid,
in Form bräunlicher Kristalle vom Smp. 290°C (Zers.).

*Beispiel 2:*

Nach dem Verfahren von Beispiel 1 können

auch die folgenden Verbindungen hergestellt werden:

2-Methyl-3,4-dihydro-4-oxo-N-(4,6,7,8-tetrahydro-4-oxo-cyclopenta-[g]-1-benzopyran-3-yl)-2H-1,2-benzothiazin-3-carboxamid-1,1-dioxid, Smp. 293-295°C,

2-Methyl-3,4-dihydro-4-oxo-N-(4-oxo-6-methyl-4H-1-benzopyran-3-yl)-2H-1,2-benzothiazin-3-carboxamid-1,1-dioxid, Smp. 272-275°C,

2-Methyl-3,4-dihydro-4-oxo-N-(4-oxo-8-methyl-4H-1-benzopyran-3-yl)-2H-1,2-benzothiazin-3-carboxamid-1,1-dioxid, Smp. 303-305°C,

2-Methyl-3,4-dihydro-4-oxo-N-(2-oxo-4-hydroxy-6-methyl-2H-1-benzopyran-3-yl)-2H-1,2-benzothiazin-3-carboxamid-1,1-dioxid, Smp. 279°C (Zers.),

2-Methyl-3,4-dihydro-4-oxo-N-(4-oxo-6-chlor-8-methyl-4H-1-benzopyran-3-yl)-2H-1,2-benzothiazin-3-carboxamid-1,1-dioxid, Smp. 292-295°C,

2-Methyl-3,4-dihydro-4-oxo-N-(4-oxo-6,7-dimethyl-4H-1-benzopyran-3-yl)-2H-1,2-benzothiazin-3-carboxamid-1-dioxid, Smp. 292-297°C,

2-Methyl-3,4-dihydro-4-oxo-N-(4-oxo-6-methoxy-4H-1-benzopyran-3-yl)-2H-1,2-benzothiazin-3-carboxamid-1,1-dioxid, Smp. 272-276°C,

2-Methyl-3,4-dihydro-4-oxo-N-(4-oxo-5,7-dimethyl-4H-1-benzopyran-3-yl)-2H-1,2-benzothiazin-3-carboxamid-1,1-dioxid, Smp. 286-291°C (Zers.),

2-Methyl-3,4-dihydro-4-oxo-N-(2-oxo-4-hydroxy-6-chlor-2H-1-benzopyran-3-yl)-2H-1,2-benzothiazin-3-carboxamid-1,1-dioxid,

2-Methyl-3,4-dihydro-4-oxo-N-(2-oxo-4-hydroxy-6,8-dichlor-2H-1-benzopyran-3-yl)-2H-1,2-benzothiazin-3-carboxamid-1,1-dioxid,

2-Methyl-3,4-dihydro-4-oxo-N-(2-oxo-4-hydroxy-4,6,7,8-tetrahydro-cyclopenta[g]-2H-1-benzopyran-3-yl)-2H-1,2-benzothiazin-3-carboxamid-1,1-dioxid, und

2-Methyl-3,4-dihydro-4-oxo-N-(2-oxo-4-hydroxy-8-methyl-2H-1-benzopyran-3-yl)-2H-1,2-benzothiazin-3-carboxamid-1,1-dioxid.

*Beispiel 3:*

8,1 g 2-Methyl-3,4-dihydro-4-oxo-2H-1,2-benzothiazin-3-carbonsäuremethylester-1,1-dioxid und 5,7 g 4-Methyl-7-aminocumarin werden in 280 ml Xylol gelöst und die Lösung zur Abdestillation des bei der Reaktion gebildeten Methanols 2,5 Stunden in einem Bad von 170°C erwärmt. Man lässt weitere 5 Stunden am Rückfluss sieden, kühlt ab und filtriert den Niederschlag ab. Dieser wird mit 100 ml 2n-Salzsäure in der Wärme digeriert mit 100 ml Chloroform versetzt und ausgeschüttelt. Der Rückstand wird abgenutscht, nacheinander mit Wasser, Äthanol, Methylenchlorid und Diäthyläther gewaschen und getrocknet. Man erhält
2-Methyl-3,4-dihydro-4-oxo-N-(2-oxo-4-methyl-2H-1-benzopyran-7-yl)-2H-1,2-benzothiazin-3-carboxamid-1,1-dioxid
vom Smp. über 285°C.

*Beispiel 4:*

Eine Lösung von 6,0 g
2-Methyl-N-(4-oxo-4H-1-benzopyran-3-yl)-4-(1-pyrrolidinyl)-2H-1,2-benzothiazin-3-carboxamid-1,1-dioxid
in 150 ml Eisessig wird auf einem Dampfbad erhitzt, und mit 150 ml n-Salzsäure versetzt. Nach 45minütigem Erhitzen wird das Reaktionsgemisch mit Eiswasser auf ein Volumen von 1,5 Liter verdünnt und filtriert, wobei in fast quantitativer Ausbeute ein Material vom Smp. 290°C (Zers.) erhalten wird. Dieses ist nach Ausweis von Mischschmelzpunkt und IR-Spektrum identisch mit der durch Umsetzung von
2-Methyl-4-oxo-3,4-dihydro-2H-1,2-benzothiazin-3-carbonsäuremethylester-1,1-dioxid
mit 3-Amino-4-oxo-4H-1-benzopyran gemäss Beispiel 1 erhaltenen Substanz, d.h. 2-Methyl-3,4-dihydro-4-oxo-N-(4-oxo-4H-1-benzopyran-3-yl)-2H-1,2-benzothiazin-3-carboxamid-1,1-dioxid
vom Smp. 290°C (Zers.).

Das Ausgangsmaterial kann z.B. folgendermassen hergestellt werden:

Eine Lösung von 2,5 g (0,025 mol) Phosgen in Toluol wird mit 50 ml trockenem Tetrahydrofuran verdünnt, hierauf auf eine Temperatur von −40°C gekühlt und schliesslich innerhalb von 30°C bei einer Temperatur von −40 bis +55°C langsam unter Rühren mit einer Lösung von 6,6 g (0,025 mol)
2-Methyl-4-(1-pyrrolidinyl)-2H-1,2-benzothiazin-1,1-dioxid
und 2,5 g (0,025 mol) Triäthylamin in 200 ml Tetrahydrofuran versetzt. Das erhaltene Reaktionsgemisch wird 12 Stunden lang bei Raumtemperatur gerührt, dann auf eine Temperatur von 10°C gekühlt und schliesslich langsam mit einer Suspension von 6,25 (0,05 mol) 3-Amino-4-oxo-4H-1-benzopyran in 100 ml Tetrahydrofuran versetzt. Man lässt die Temperatur des erhaltenen Reaktionsgemisches auf Raumtemperatur ansteigen und das Reaktionsgemisch 72 Stunden nachrühren, giesst auf 1 Liter Eiswasser, nutscht ab und kristallisiert aus Tetrahydrofuran. Man erhält das reine, kristalline
2-Methyl-N-(4-oxo-4H-1-benzopyran-3-yl)-4-(1-pyrrolidinyl)-2H-1,2-benzothiazin-3-carboxamid-1,1-dioxid.

In analoger Weise kann man ausgehend von 2-Methyl-N-(6-chlor-7-methyl-4-oxo-4H-1-benzopyran-3-yl)-4-(1-pyrrolidinyl)-1,2-benzothiazin-3-carboxamid-1,1-dioxid das 2-methyl-3,4-dihydro-4-oxo-N-(6-chlor-7-methyl-4-oxo-4H-1-benzopyran-3-yl)-2H-1,2-benzothiazin-3-carboxamid-1,1-dioxid herstellen.

*Beispiel 5:*

Zu einer Lösung von 4 g rohem 2-Methoxycarbonyl-N-methyl-N-[N'-(4-oxo-4H-1-benzopyran-3-yl)carbamoylmethyl]benzolsulfamid in 50 ml absolutem Dimethylformamid fügt man unter Stickstoff etwa 1 g Natriummethylat hinzu und erwärmt vorsichtig auf 80°C. Dann wird im Vakuum zur Trockne eingedampft und der Eindampfrückstand mit Wasser versetzt und mit 2n-Salzsäure neutralisiert. Das dabei anfallende rohe 2-Methyl-3,4-dihydro-4-oxo-N-(4-oxo-4H-1-benzopyran-3-yl)-2H-1,2-benzothiazin-3-carboxamid-1,1-dioxid wird abgetrennt und auf analoge Weise wie in Beispiel 1 sowie durch Chromatographie an Silicagel gereinigt. Es schmilzt bei 280°C.

Das Ausgangsmaterial kann z.B. wie folgt erhalten werden:

Zu 8 g 3-Aminochromon in 200 ml Toluol fügt man 10 ml Chloroform und erwärmt auf 40°C. Dann kühlt man auf 20°C und versetzt unter Rühren mit 10 ml N,N-Diisopropyläthylamin und anschliessend bei 10°C innerhalb von 5 Minuten mit 4,8 ml Bromacetylchlorid in 20 ml Toluol. Nach beendeter Zugabe wird 1 Stunde bei Raumtemperatur nachgerührt. Dann wird das gebildete Kristallisat abgesaugt, mit Wasser gewaschen und aus Äthanol/Petroläther umkristallisiert. Man erhält so 11,8 g 2-Brom-N-(4-oxo-1-benzopyran-3-yl)acetamid vom F. 198°C.

Eine Lösung von 7,5 g 2-Brom-N-(4-oxo-1-benzopyran-3-yl)acetamid und 5,5 g Saccharin-Natrium in 100 ml absolutem Dimethylformamid wird 5 Stunden auf 100°C erwärmt. Dann wird auf Raumtemperatur gekühlt, das gebildete Kristallisat abfiltriert und mit Äthanol gewaschen. Man erhält so 8,6 g 2-(1,1,3-Trioxo-2,3-dihydrobenzothiazol-2-yl)-N-(4-oxo-1-benzopyran-3-yl)acetamid vom F. 280 bis 281°C.

Zu einer Lösung von 3,8 g des obigen Produktes in 100 ml Dimethylformamid fügt man 0,7 g Natriummethylat und erwärmt kurz auf 80°C. Dann kühlt man auf 30°C und versetzt mit 2 g Methyljodid. Man lässt 2 Studen bei 30°C nachrühren. Anschliessend erwärmt man langsam auf etwa 70°C und dampft im Vakuum auf die Hälfte des Volumens ein. Das im Reaktionsgemisch enthaltene 2-Methoxycarbonyl-N-methyl-N-[N'-(4-oxo-4H-1-benzopyran-3-yl)carbamoylmethyl]benzolsulfamid wird direkt weiterverarbeitet.

*Beispiel 6:*

Zu 4,0 g 2-Methyl-N-[(2-hydroxy)benzoylmethyl]-3,4-dihydro-2H-1,2-benzothiazin-3-carboxamid-1,1-dioxid und 1,4 g Natriumformiat fügt man unter Rühren bei Raumtemperatur 10 ml des gemischten Anhydrids von Ameisen- und Essigsäure hinzu. Man lässt 1 Stunde rühren und erwärmt anschliessend 3 Stunden auf etwa 100°C. Dann kühlt man auf Raumtemperatur ab und versetzt mit 200 ml kaltem Wasser. Das dabei anfallende rohe 2-Methyl-4-oxo-N-(4-oxo-4H-1-benzopyran-3-yl)-3,4-dihydro-2H-1,2-benzothiazin-1,1-dioxid wird abgetrennt und auf analoge Weise wie in Beispiel 1 beschrieben und durch anschliessende Chromatographie an Silicagel gereinigt. Es schmilzt bei 280°C.

Das Ausgangsmaterial kann z.B. ausgehend von α-Amino-2-hydroxyacetophenon und 2-Methyl-4-oxo-3,4-dihydro-1,2-benzothiazin-3-carbonsäuremethylester-1,1-dioxid nach dem in Beispiel 1 beschriebenen Verfahren erhalten werden (Smp. 256 bis 258°C, aus Dimethylformamid/Äthanol = 1:10).

*Beispiel 7:*

5g 2-Methyl-4-oxo-(N-4-oxo-4H-1,4-benzopyran-3-yl)-3,4-dihydro-2H-1,2-benzothiazin-3-carboxamid-1,1-dioxid werden in einem Gemisch aus 125 ml 0,1 n-Natronlauge und 100 ml Methanol bei 50°C gelöst. Man kühlt auf 5°C ab und sammelt die gelben Kristalle des Natriumsalzmonohydrates von 2-Methyl-4-oxo-N-(4-oxo-4H-1,4-benzopyran-3-yl)-3,4-dihydro-2H-1,2-benzothiazin-3-carboxamid-1,1-dioxid von Smp. 240 bis 245°C (Zers.)

*Beispiel 8:*

5 g Natriumsalzmonohydrat des 2-Methyl-4-oxo-N-(4-oxo-4H-1,4-benzopyran-3-yl)-3,4-dihydro-2H-1,2-benzothiazin-3-carboxamid-1,1-dioxides werden in 900 ml Methanol und 400 ml Wasser gelöst und mit einer Lösung von 1,65 g Zinksulfatdihydrat in 50 ml Wasser versetzt. Man erhält das Zinksalz des 2-Methyl-4-oxo-N-(4-oxo-4H-1,4-benzopyran-3-yl)-3,4-dihydro-2H-1,2-benzothiazin-3-carboxamid-1,1-dioxides vom Smp. 280°C (Zers.).

*Beispiel 9:*

5g Natriumsalz-Monohydrat des 2-Methyl-4-oxo-N-(4-oxo-4H-1,4-benzopyran-3-yl)-3,4-dihydro-2H-1,2-benzothiazin-3-carboxamid-1,1-dioxides werden in 1000 ml Methanol und 200 ml Wasser gelöst und mit einer Lösung von 1,2 g Kupferacetat versetzt. Man erhält das Kupfersalz des 2-Methyl-4-oxo-N-(4-oxo-4H-1,4-benzopyran-3-yl)-3,4-dihydro-2H-1,2-benzothiazin-3-carboxamid-1,1-dioxides vom Smp. über 300°C.

*Beispiel 10:*

12,7 g 4-Oxo-3,4-dihydro-2H-1,2-benzothiazin-3-carbonsäuremethylester-1,1-dioxid und 9,7 g 3-Amino-4-oxo-4H-1-benzopyran werden in 500 ml Xylol 5 Minuten lang mit Stickstoff begast, langsam zum Sieden erhitzt und 15 Stunden lang unter gelegentlichem Nachfüllen von Lösungsmittel destilliert. Man lässt auf 50°C

abkühlen, saugt ab und wäscht mit Äthanol. Nach dem Trocknen erhält man das
4-Oxo-N-(4-oxo-4H-1,4-benzopyran-3-yl)-3,4-dihydro-2H-1,2-benzothiazin-3-carboxamid-1,1-dioxid
vom Smp. 288 bis 290°C.

Durch Umsetzung dieser Verbindung mit einem reaktionsfähigen Äthanolester, z.B. mit Äthyliodid, erhält man das
2-Äthyl-4-oxo-N-(4-oxo-4H-1,4-benzopyran-3-yl)-3,4-dihydro-2H-1,2-benzothiazin-3-carboxamid-1,1-dioxid
und durch Umsetzung und einem reaktionsfähigen Allylalkoholester, z.B. mit Allylbromid, das
2-Allyl-4-oxo-N-(4-oxo-4H-1,4-benzopyran-3-yl)-3,4-dihydro-2H-1,2-benzothiazin-3-carboxamid-1,1-dioxid, Smp. 200°C.

*Beispiel 11:*

Tabletten enthaltend 25 mg
2-Methyl-3,4-dihydro-4-oxo-N-(4H-1-benzo-pyran-3-yl)-2H-1,2-benzothiazin-3-carboxamid-1,1-dioxid
können z.B. wie folgt hergestellt werden:

*Zusammensetzung* (für 10000 Tabletten):

| | |
|---|---|
| 2-Methyl-3,4-dihydro-4-oxo-N-(4H-1-benzo-pyran-3-yl)-2H-1,2-benzothiazin-3-carboxamid-1,1-dioxid (Wirkstoff) | 250 g |
| Lactose | 460 g |
| Maisstärke | 650 g |
| Polyvinylpyrrolidon | 20 g |
| Magnesiumstearat | 10 g |
| kolloidales Siliciumoxid | 10 g |
| Wasser | q.s. |

Der Wirkstoff, die Lactose und 450 g der Maisstärke werden gemischt und mit einer wässrigen Lösung von Polyvinylpyrrolidon befeuchtet. Das Gemisch wird granuliert und getrocknet, und mit dem Magnesiumstearat, dem kolloidalen Siliciumoxid und dem Rest der Maisstärke versetzt. Das Gemisch wird durch ein Sieb getrieben, gemischt und zu Tabletten von 140 mg Gewicht (Durchmesser: 7 mm) verpresst.

*Beispiel 12:*

In analoger Weise wie in Beispiel 11 beschrieben können auch Tabletten enthaltend jeweils 25 mg

2-Methyl-3,4-dihydro-4-oxo-N-(4,6,7,8-tetrahydro-4-oxocyclopenta-[g]-1-benzopyran-3-yl)-2H-1,2-benzothiazin-3-carboxamid-1,1-dioxid,

2-Methyl-3,4-dihydro-4-oxo-N-(4-oxo-6-methyl-4H-1-benzopyran-3-yl)-2H-1,2-benzothiazin-3-carboxamid-1,1-dioxid,

2-Methyl-3,4-dihydro-4-oxo-N-(4-oxo-8-methyl-4H-1-benzopyran-3-yl)-2H-1,2-benzothiazin-3-carboxamid-1,1-dioxid,

2-Methyl-3,4-dihydro-4-oxo-N-(2-oxo-4-hydroxy-6-methyl-2H-1-benzopyran-3-yl)-2H-1,2-benzothiazin-3-carboxamid-1,1-dioxid,

2-Methyl-3,4-dihydro-4-oxo-N-(4-oxo-6,7-dimethyl-4H-1-benzopyran-3-yl)-2H-1,2-benzothiazin-3-carboxamid-1-dioxid,

2-Methyl-3,4-dihydro-4-oxo-N-(4-oxo-6-methoxy-4H-1-benzopyran-3-yl)-2H-1,2-benzothiazin-3-carboxamid-1,1-dioxid,

2-Methyl-3,4-dihydro-4-oxo-N-(4-oxo-5,7-dimethyl-4H-1-benzopyran-3-yl)-2H-1,2-benzothiazin-3-carboxamid-1,1-dioxid,

2-Methyl-3,4-dihydro-4-oxo-N-(2-oxo-4-hydroxy-6-chlor-2H-1-benzopyran-3-yl)-2H-1,2-benzothiazin-3-carboxamid-1,1-dioxid,

2-Methyl-3,4-dihydro-3-oxo-N-(2-oxo-4-hydroxy-6,8-dichlor-2H-1-benzopyran-3-yl)-2H-1,2-benzothiazin-3-carboxamid-1,1-dioxid,

2-Methyl-3,4-dihydro-4-oxo-N-(2-oxo-4-hydroxy-4,6,7,8-tetrahydrocyclopenta-[g]-2H-1-benzopyran-3-yl)-2H-1,2-benzothiazin-3-carboxamid-1,1-dioxid,

2-Methyl-3,4-dihydro-4-oxo-N-(2-oxo-4-hydroxy-8-methyl-2H-1-benzopyran-3-yl)-2H-1,2-benzothiazin-3-carboxamid-1,1-dioxid, und

2-Methyl-3,4-dihydro-4-oxo-N-(2-oxo-4-methyl-2H-1-benzopyran-7-yl)-2H-1,2-benzothiazin-3-carboxamid-1,1-dioxid
hergestellt werden.

*Beispiel 13:*

In analoger Weise wie in Beispiel 11 beschrieben können auch Tabletten enthaltend 25 mg

2-Methyl-N-(6-chlor-8-methyl-4-oxo-4H-1-benzopyran-3-yl)-4-oxo-3,4-dihydro-2H-1,2-benzothiazin-3-carboxamid-1,1-dioxid,

2-Methyl-3,4-dihydro-4-oxo-N-(6-chlor-7-methyl-4-oxo-4H-1-benzopyran-3-yl)-2H-1,2-benzothiazin-3-carboxamid-1,1-dioxid,

Natriumsalzmonohydrat von 2-Methyl-4-oxo-N-(4-oxo-4H-1,4-benzopyran-3-yl)-3,4-dihydro-2H-1,2-benzothiazin-3-carboxamid-1-1-dioxid,

2-Methyl-3,4-dihydro-4-oxo-N-(4,6,7,8-tetrahydro-4-oxocyclopenta-[g]-1-benzopyran-3-yl)-2H-1,2 benzothiazin-3-carboxamid-1,1-dioxidnatriumsalzhydrat,

2-Methyl-3,4-dihydro-4-oxo-N-(4-oxo-6,7-dimethyl-4H-1-benzopyran-3-yl)-2H-1,2-benzothiazin-3-carboxamid-1-dioxidnatriumsalzhydrat,

2-Methyl-3,4-dihydro-4-oxo-N-(4-oxo-6-methoxy-4H-1-benzopyran-3-yl)-2H-1,2-benzothiazin-3-carboxamid-1,1-dioxidnatriumsalzhydrat,

2-Methyl-3,4-dihydro-4-oxo-N-(4-oxo-5,7-dimethyl-4H-1-benzopyran-3-yl)-2H-1,2-benzothiazin-3-carboxamid-1,1-dioxidnatriumsalzhydrat,

2-Methyl-3,4-dihydro-4-oxo-N-(4-oxo-8-methyl-4H-1-benzopyran-3-yl)-2H-1,2-benzothiazin-3-carboxamid-1,1-dioxid,

2-Methyl-3,4-dihydro-4-oxo-N-(4-oxo-8-methyl-4H-1-benzopyran-3-yl)-2H-1,2-benzothiazin-3-carboxamid-1,1-dioxidnatriumsalzhydrat,

2-Methyl-3,4-dihydro-4-oxo-N-(4-oxo-6-methyl-4H-1-benzopyran-3-yl)-2H-1,2-benzothiazin-3-carboxamid-1,1-dioxidnatriumsalzhydrat,

4-Oxo-N-(4-oxo-4H-1,4-benzopyran-3-yl)-3,4-dihydro-2H-1,2-benzothiazin-3-carboxamid-1,1-dioxid,

2-Äthyl-4-oxo-N-(4-oxo-4H-1,4-benzopyran-3-yl)-3,4-dihydro-2H-1,2-benzothiazin-3-carboxamid-1,1-dioxid, und

2-Allyl-4-oxo-N-(4-oxo-4H-1,3-benzopyran-3-yl)-3,4-dihydro-2H-1,2-benzothiazin-3-carboxamid-1,1-dioxid hergestellt werden.

*Beispiel 14:*

In analoger Weise wie in Beispiel 7 beschrieben kann man ferner herstellen:

2-Methyl-3,4-dihydro-4-oxo-N-(4-oxo-6-methoxy-4H-1-benzopyran-3-yl)-2H-1,2-benzothiazin-3-carboxamid-1,1-dioxidnatriumsalzhydrat, Smp. 235-239°C,

2-Methyl-3,4-dihydro-4-oxo-N-(4-oxo-5,7-dimethyl-4H-1-benzopyran-3-yl)-2H-1,2-benzothiazin-3-carboxamid-1,1-dioxidnatriumsalzhydrat, Smp. 234-236°C,

2-Methyl-3,4-dihydro-4-oxo-N-(4-oxo-8-methyl-4H-1-benzopyran-3-yl)-2H-1,2-benzothiazin-3-carboxamid-1,1-dioxidnatriumsalzhydrat, Smp. 239-241°C,

2-Methyl-3,4-dihydro-4-oxo-N-(4-oxo-6-methyl-4H-1-benzopyran-3-yl)-2H-1,2-benzothiazin-3-carboxamid-1,1-dioxidnatriumsalzhydrat, Smp. 235-239°C,

2-Methyl-3,4-dihydro-4-oxo-N-(4-oxo-4,6,7,8-tetrahydrocyclopenta-[g]-1-benzopyran-3-yl)-2H-1,2-benzothiazin-3-carboxamid-1,1-dioxidnatriumsalzmonohydrat, Smp. 240-241°C,

2-Methyl-3,4-dihydro-4-oxo-N-(4-oxo-6,7-dimethyl-4H-1,2-benzopyran-3-yl)-2H-1,2-benzothiazin-3-carboxamid-1-dioxidnatriumsalzhydrat, Smp. 260-261°C, und

2-Methyl-N-(6-chlor-7-methyl-4-oxo-4H-1-benzopyran-3-yl)-4-oxo-3,4-dihydro-2H-1,2-benzothiazin-3-carboxamid-1,1-dioxid-natriumsalzmonohydrat, Smp. 239-241°C.

## Patentansprüche

1. Ein substituiertes 3,4-Dihydro-2H-1,2-benzothiazin-1,1-dioxyd der Formel

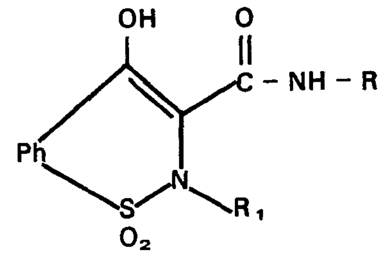

(I)

worin Ph einen gegebenenfalls durch Niederalkyl, Niederalkoxy, Niederalkanoyl, Halogen, Nitro und/oder Trifluormethyl substituierten 1,2-Phenylenrest bedeutet, R einen gegebenenfalls durch Niederalkyl, Niederalkoxy, Halogen, Niederalkanoyloxy, Hydroxy, Benzoyloxyreste, gegebenenfalls durch Niederalkyl oder Niederalkanoyl substituiertes Amino oder an zwei benachbarten Kohlenstoffatomen durch Niederalkylen oder Niederalkylendioxy substituierten Benzopyronrest bedeutet, und $R_1$ Wasserstoff, Niederalkyl, Niederalkenyl, Hydroxyniederalkyl, Niederalkoxyniederalkyl oder einen Phenylniederalkylrest darstellt, wobei in den genannten Gruppen Phenyl durch Niederalkyl, Niederalkoxy, Halogen, Trifluormethyl und/oder Nitro substituiert sein kann, oder ein Salz davon.

2. Eine Verbindung gemäss Anspruch 1, worin Ph einen unsubstituierten oder durch Niederalkyl mit bis zu 4 Kohlenstoffatomen, Niederalkoxy mit bis zu 4 Kohlenstoffatomen, Halogen bis Atomnummer 35, Nitro oder Trifluormethyl substituierten 1,2-Phenylenrest bedeutet, einen in 3-, 4-, 6- oder 7-Stellung gebundenen 2-Oxo-2H-1-benzopyranylrest oder in 3- oder 6- oder 7-Stellung gebundenen 4-Oxo-4H-1-benzopyranylrest darstellt, der unsubstituiert oder durch Niederalkyl mit bis zu 4 Kohlenstoffatomen, Niederalkoxy mit bis zu 4 Kohlenstoffatomen, Halogen bis Atomnummer 35, Niederalkanoyloxy mit bis zu 4 Kohlenstoffatomen, Hydroxy, Amino, N-Mono- oder N,N-Diniederalkylamino mit bis zu 4 Kohlenstoffatomen im Alkylteil, Niederalkanoylamino mit bis zu 4 Kohlenstoffatomen und/oder an zwei benachbarten Kohlenstoffatomen durch Niederalkylen oder Niederalkylendioxy mit bis zu 4 Kohlenstoffatomen substituiert sein kann, und $R_1$ Wasserstoff oder Niederalkyl mit bis zu 4 Kohlenstoffatomen bedeutet, oder ein Salz davon mit einer Base.

3. Eine Verbindung gemäss Anspruch 1 der Formeln Ib und Ic

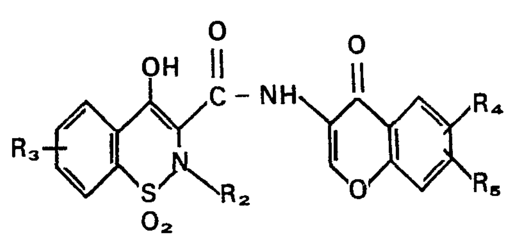

(Ib)

und

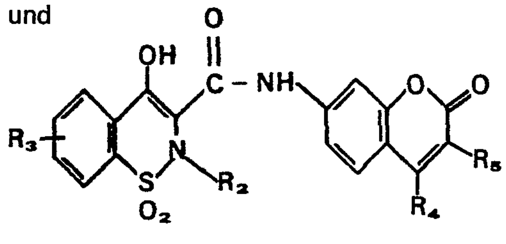

(Ic)

worin $R_2$ Wasserstoff oder Niederalkyl mit bis zu 4 Kohlenstoffatomen bedeutet, $R_3$ Wasserstoff, Niederalkyl mit bis zu 4 Kohlenstoffatomen, Niederalkoxy mit bis zu 4 Kohlenstoffatomen, Halogen bis Atomnummer 35, Trifluormethyl oder Nitro bedeutet, und $R_4$ und $R_5$ unabhängig voneinander Wasserstoff, Niederalkyl mit bis zu 4 Kohlenstoffatomen oder Niederalkoxy mit bis zu 4 Kohlenstoffatomen bedeuten oder gemeinsam einen an benachbarte Kohlenstoffatome gebundenen 3- oder 4-gliedrigen Niederalkylen- oder Niederalkylendioxyrest mit bis zu 4 Kohlenstoffatomen, darstellen oder in Formel Ic $R_4$ Hydroxy und $R_5$ Wasserstoff oder Niederalkyl mit bis zu 4 Kohlenstoffatomen bedeutet, oder ein Salz davon mit Base.

4. Eine Verbindung gemäss Anspruch 3, worin $R_2$ Niederalkyl mit bis zu 4 Kohlenstoffatomen bedeutet, $R_3$ Wasserstoff bedeutet, und $R_4$ und $R_5$ unabhängig voneinander Wasserstoff, Niederalkyl oder Niederalkoxy mit bis zu 4 Kohlenstoffatomen bedeuten, oder ein Salz davon mit Base.

5. Eine Verbindung der Gruppe bestehend aus 2-Methyl-3,4-dihydro-4-oxo-N-(4-oxo-4H-1-benzopyran-3-yl)-2H-1,2-benzothiazin-3-carboxamid-1,1-dioxid und seinen Salzen mit Basen.

6. Eine Verbindung gemäss Anspruch 1 bestehend aus

2-Methyl-3,4-dihydro-4-oxo-N-(2-oxo-4-methyl-2H-1-benzopyran-7-yl)-2H-1,2-benzothiazin-3-carboxamid-1,1-dioxid,

2-Methyl-3,4-dihydro-4-oxo-N-(4-oxo-4,6,7,8-tetrahydrocyclopenta-[g]-1-benzopyran-3-yl)-2H-1,2-benzothiazin-3-carboxamid-1,1-dioxid,

2-Methyl-3,4-dihydro-4-oxo-N-(2-oxo-4-hydroxy-6-methyl-2H-1-benzopyran-3-yl)-2H-1,2-benzothiazin-3-carboxamid-1,1-dioxid,

2-Methyl-3,4-dihydro-4-oxo-N-(4-oxo-6,7-dimethyl-4H-1-benzopyran-3-yl)-2H-1,2-benzothiazin-3-carboxamid-1,1-dioxid,

2-Methyl-3,4-dihydro-4-oxo-N-(4-oxo-6-methoxy-4H-1-benzopyran-3-yl)-2H-1,2-benzothiazin-3-carboxamid-1,1-dioxid,

2-Methyl-3,4-dihydro-4-oxo-N-(4-oxo-5,7-dimethyl-4H-1-benzopyran-3-yl)-2H-1,2-benzothiazin-3-carboxamid-1,1-dioxid,

2-Methyl-3,4-dihydro-4-oxo-N-(4-oxo-8-methyl-4H-1-benzopyran-3-yl)-2H-1,2-benzothiazin-1,1-dioxid,

2-Methyl-3,4-dihydro-4-oxo-N-(4-oxo-6-methyl-4H-1-benzopyran-3-yl)-2H-1,2-benzothiazin-3-carboxamid-1,1-dioxid,

2-Methyl-3,4-dihydro-4-oxo-N-(2-oxo-4-hydroxy-6-chlor-2H-1-benzopyran-3-yl)-2H-1,2-benzothiazin-3-carboxamid-1,1-dioxid,

2-Methyl-3,4-dihydro-4-oxo-N-(2-oxo-4-hydroxy-6,8-dichlor-2H-1-benzopyran-3-yl)-2H-1,2-benzothiazin-3-carboxamid-1,1-dioxid,

2-Methyl-3,4-dihydro-4-oxo-N-(2-oxo-4-hydroxy-4,6,7,8-tetrahydrocyclopenta-[g]-2H-1-benzopyran-3-yl)-2H-1,2-benzothiazin-3-carboxamid-1,1-dioxid,

2-Methyl-3,4-dihydro-4-oxo-N-(2-oxo-4-hydroxy-8-methyl-2H-1-benzopyran-3-yl)-2H-1,2-benzothiazin-3-carboxamid-1,1-dioxid,

2-Methyl-3,4-dihydro-4-oxo-N-(4-oxo-6-chlor-8-methyl-4H-1-benzopyran-3-yl)-2H-1,2-benzothiazin-3-carboxamid-1,1-dioxid,

3,4-Dihydro-4-oxo-N-(4-oxo-4H-1-benzopyran-3-yl)-2H-1,2-benzothiazin-3-carboxamid-1,1-dioxid,

2-Äthyl-3,4-dihydro-4-oxo-4H-1-benzopyran-3-yl)-2H-1,2-benzothiazin-3-carboxamid-1,1-dioxid, und

2-Allyl-3,4-dihydro-4-oxo-N-(4-oxo-4H-1-benzopyran-3-yl)-2H-1,2-benzothiazin-carboxamid-1,1-dioxid oder Salzen derselben.

7. Eine Verbindung gemäss einem der Ansprüche 1-6 in Form des Natriumsalzes oder des Zinksalzes.

8. Eine Verbindung gemäss einem der Ansprüche 1-7 mit antiinflammatorischer Wirkung.

9. Pharmazeutische Präparate enthaltend eine Verbindung gemäss einem der Ansprüche 1-8 gegebenenfalls in Form eines pharmazeutisch verwendbaren Salzes mit einer Base zusammen mit üblichen Hilfs- und/oder Trägerstoffen.

10. Eine Verbindung gemäss einem der Ansprüche 1-8, gegebenenfalls in Form eines pharmazeutisch verwendbaren Salzes mit einer Base zur therapeutischen Behandlung des menschlichen oder tierischen Körpers.

11. Eine Verbindung gemäss einem der Ansprüche 1-8 zur Anwendung gemäss Anspruch 9 als Antiinflammatorikum.

12. Verwendung einer Verbindung gemäss einem der Ansprüche 1-8 zur Herstellung eines Arzneimittels, insbesondere eines Antiinflammatorikums.

13. Eine Verbindung gemäss einem der Ansprüche 1-8 zur Bekämpfung von Krankheiten, insbesondere entzündlicher Erkrankungen.

14. Verfahren zur Herstellung neuer 3,4-Dihydro-2H-1,2-benzothiazin-1,1-dioxyde der Formel I

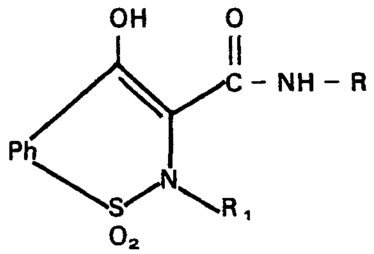

(I)

worin Ph, R und $R_1$ wie in Anspruch 1 definiert sind, und ihre Salze, dadurch gekennzeichnet, dass man eine Verbindung der Formel

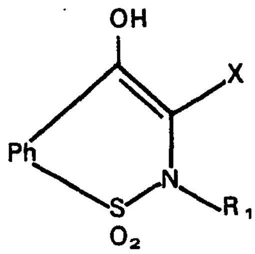

(II)

wobei X eine gegebenenfalls funktionell abgewandelte Carboxygruppe bedeutet, oder ein Salz davon, mit einer Verbindung der Formel $R-NH_2$ (III) oder mit einem reaktionsfähigen Derivat davon umsetzt, oder eine Verbindung der Formel

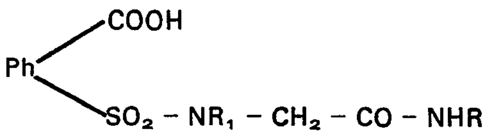

(IV)

oder ein funktionelles Carboxyderivat und/oder Salz davon zu einer Verbindung der Formel I cyclisiert, oder eine Verbindung der Formel

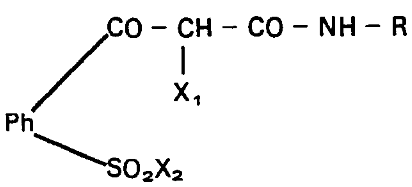

(V)

worin einer der Reste $X_1$ und $X_2$ eine Gruppe der Formel $-NHR_1$ und der andere eine gegebenenfalls verätherte oder reaktionsfähig veresterte Hydroxygruppe oder Aminogruppe X bedeutet, intramolekular cyclisiert, oder in einer Verbindung der Formel VI

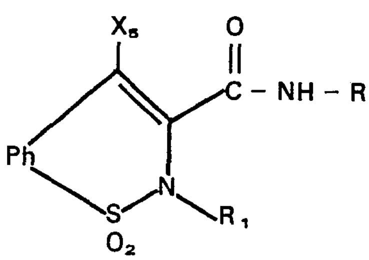

(VI)

worin $X_5$ einen in Hydroxy überführbaren Rest bedeutet, oder in einem Salz davon $X_5$ in Hydroxy überführt oder in einer Verbindung der Formel VII

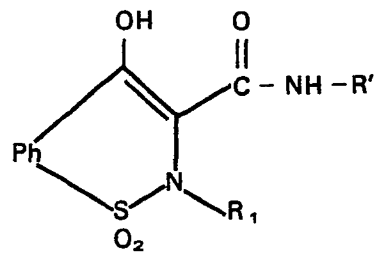

(VII)

worin R' eine zu dem gegebenenfalls substituierten Benzopyronrest R cyclisierbaren Rest bedeutet, oder in einem Salz davon R' zu R ringschliesst und, wenn erwünscht, ein verfahrensgemäss erhältliches Salz in die freie Verbindung oder in ein anderes Salz oder ein verfahrensgemäss erhältliches Salz in die freie Verbindung umwandelt.

**Claims**

1. A substituted 3,4-dihydro-2H-1,2-benzothiazine-1,1-dioxide of the formula

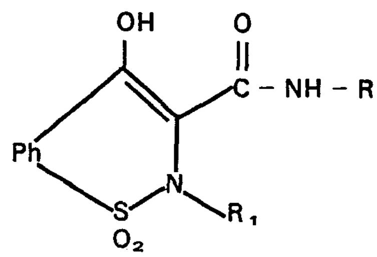

(I)

wherein Ph is a 1,2-phenylene radical, optionally substituted by lower alkyl, lower alkoxy, lower alkanoyl, halogen, nitro and/or trifluoromethyl, R is a benzopyrone radical, optionally substituted by lower alkyl, lower alkoxy, halogen, lower alkanoyloxy, hydroxy, benzoyloxy, amino, optionally substituted by lower alkyl or lower alkanoyl, or at two adjacent carbon atoms by lower alkylene or lower alkylenedioxy, and $R_1$ is hydrogen, lower alkyl, lower alkenyl, hydroxy-lower alkyl, lower alkoxy-lower alkyl or phenyl-lower alkyl, whilst phenyl, in the above groups, can be substituted by lower alkyl, lower alkoxy, halogen, trifluoromethyl and/or nitro, or a salt thereof.

2. A compound according to claim 1, wherein Ph is a 1,2-phenylene radical, unsubstituted or substituted by lower alkyl of 1 to 4 carbon atoms, lower alkoxy of up to 4 carbon atoms, halogen with an atomic number up to 35, nitro or trifluoromethyl, R is a 2-oxo-2H-1-benzopyrannyl radical which is bonded in the 3-, 4-, 6- or 7-position or a 4-oxo-4H-1-benzopyrannyl radical which is bonded in the 3-, 6- or 7-position, which radical can be unsubstituted or substituted by lower alkyl of up to 4 carbon atoms, lower alkoxy of up to 4 carbon atoms, halogen with an atomic number up to 35, lower alkanoyloxy of up to 4 carbon atoms, hydroxy, amino, N-mono- or N,N-di-lower alkylamino containing up to 4 carbon atoms in the alkyl moiety, lower alkanoylamino of up to 4 carbon atoms, and/or at two adjacent carbon atoms by lower alkylene or lower alkylenedioxy of up to 4 carbon atoms, and $R_1$ is

hydrogen or lower alkyl of up to 4 carbon atoms, or a salt thereof with a base.

3. A compound according to claim 1, of the formulae Ib and Ic

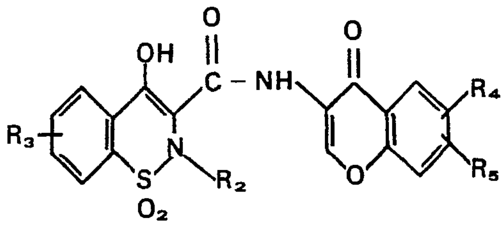

(Ib)

and

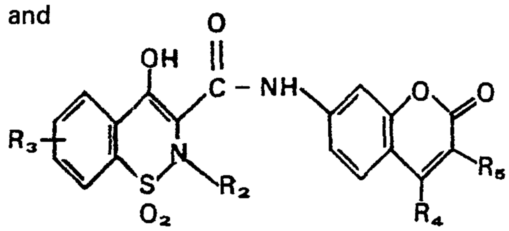

(Ic)

wherein $R_2$ is hydrogen or lower alkyl of up to 4 carbon atoms, $R_3$ is hydrogen, lower alkyl of up to 4 carbon atoms, lower alkoxy of up to 4 carbon atoms, halogen with an atomic number up to 35, trifluoromethyl or nitro, and each of $R_4$ and $R_5$ independently is hydrogen, lower alkyl of up to 4 carbon atoms or lower alkoxy of up to 4 carbon atoms, or together are a 3- or 4-membered lower alkylene or lower alkylenedioxy radical of up to 4 carbon atoms which is bonded to adjacent carbon atoms, or in formula Ic, $R_4$ is hydroxy and $R_5$ is hydrogen or lower alkyl of up to 4 carbon atoms, or a salt thereof with a base.

4. A compoud according to claim 3, wherein $R_2$ is lower alkyl of up to 4 carbon atoms, $R_3$ is hydrogen, and each of $R_4$ and $R_5$ independently is hydrogen, lower alkyl or lower alkoxy of up to 4 carbon atoms, or a salt thereof with a base.

5. A compound selected from the group consisting of
2-methyl-3,4-dihydro-4-oxo-N-(4-oxo-4H-1-benzopyran-3-yl)-2H-1,2-benzothiazine-3-carboxamide-1,1-dioxide,
and the salts thereof with a base.

6. A compound according to claim 1, selected from the group consisting of

2-Methyl-3,4-dihydro-4-oxo-N-(2-oxo-4-methyl-2H-1-benzopyran-7-yl)-2H-1,2-benzothiazine-3-carboxamide-1,1-dioxide,

2-Methyl-3,4-dihydro-4-oxo-N-(4-oxo-4,6,7,8-tetrahydro-cyclopenta-[g]-1-benzopyran-3-yl)-2H-1,2-benzothiazine-3-carboxamide-1,1-dioxide,

2-Methyl-3,4-dihydro-4-oxo-N-(2-oxo-4-hydroxy-6-methyl-2H-1-benzopyran-3-yl)-2H-1,2-benzothiazine-3-carboxamide-1,1-dioxide,

2-Methyl-3,4-dihydro-4-oxo-N-(4-oxo-6,7-dimethyl-4H-1-benzopyran-3-yl)-2H-1,2-benzothiazine-3-carboxamide-1,1-dioxide,

2-Methyl-3,4-dihydro-4-oxo-N-(4-oxo-6-methoxy-4H-1-benzopyran-3-yl)-2H-1,2-benzothiazine-3-carboxamide-1,1-dioxide,

2-Methyl-3,4-dihydro-4-oxo-N-(4-oxo-5,7-dimethyl-4H-1-benzopyran-3-yl)-2H-1,2-benzothiazine-3-carboxamide-1,1-dioxide,

2-Methyl-3,4-dihydro-4-oxo-N-(4-oxo-8-methyl-4H-1-benzopyran-3-yl)-2H-1,2-benzothiazine-3-carboxamide-1,1-dioxide,

2-Methyl-3,4-dihydro-4-oxo-N-(4-oxo-6-methyl-4H-1-benzopyran-3-yl)-2H-1,2-benzothiazine-3-carboxamide-1,1-dioxide,

2-Methyl-3,4-dihydro-4-oxo-N-(2-oxo-4-hydroxy-6-chlor-2H-1-benzopyran-3-yl)-2H-1,2-benzothiazine-3-carboxamide-1,1-dioxide,

2-Methyl-3,4-dihydro-4-oxo-N-(2-oxo-4-hydroxy-6,8-dichlor-2H-1-benzopyran-3-yl)-2H-1,2-benzothiazine-3-carboxamide-1,1-dioxide,

2-Methyl-3,4-dihydro-4-oxo-N-(2-oxo-4-hydroxy-4,6,7,8-tetrahydrocyclopenta-[g]-2H-1-benzopyran-3-yl)-2H-1-benzopyran-3-yl)-2H-1,2-benzothiazine-3-carboxamide-1,1-dioxide,

2-Methyl-3,4-dihydro-4-oxo-N-(2-oxo-4-hydroxy-8-methyl-2H-1-benzopyran-3-yl)-2H-1,2-benzothiazine-3-carboxamide-1,1-dioxide,

2-Methyl-3,4-dihydro-4-oxo-N-(4-oxo-6-chloro-8-methyl-4H-1-benzopyran-3-yl)-2H-1,2-benzothiazine-3-carboxamide-1,1-dioxide,

3,4-Dihydro-4-oxo-N-(4-oxo-4H-1-benzopyran-3-yl)-2H-1,2-benzothiazine-3-carboxamide-1,1-dioxide,

2-Ethyl-3,4-dihydro-4-oxo-N-(4-oxo-4H-1-benzopyran-3-yl)-2H-1,2-benzothiazine-3-carboxamide-1,1-dioxide, and

2-Allyl-3,4-dihydro-4-oxo-N-(4-oxo-4H-1-benzopyran-3-yl)-2H-1,2-benzothiazine-3-carboxamide-1,1-dioxide,

or a salt thereof.

7. A compound according to any one of claims 1 to 6, in the form of the sodium salt or zinc salt.

8. A compound according to any one of claims 1 to 7, with anti-inflammatory action.

9. A pharmaceutical preparation containing a compound according to any one of claims 1 to 8, optionally in the form of a pharmaceutically acceptable salt with a base, together with conventional adjuvants and/or carriers.

10. A compound according to any one of claims 1 to 8, optionally in the form of a pharmaceutically acceptable salt with a base for the therapeutic treatment of humans or animals.

11. A compound according to any one of claims 1 to 8 for use according to claim 9 as anti-inflammatory agent.

12. Use of a compound according to any one of claims 1 to 8 for the preparation of a medicament, especially of an anti-inflammatory agent.

13. A compound according to any one of claims 1 to 8 for combating diseases, in particular inflammatory conditions.

14. A process for the production of a novel 3,4-dihydro-2H-1,2-benzothiazine-1,1-dioxide of the formula I

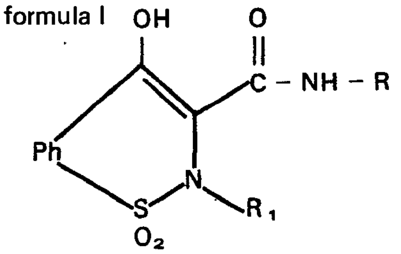

(I)

wherein Ph, R and $R_1$ are as defined in claim 1, or a salt thereof, which process comprises reacting a compound of the formula

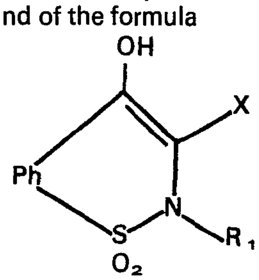

(II)

wherein X is a free or functionally modified carboxyl group, or a salt thereof, with a compound of the formula

$$R-NH_2 \quad (III)$$

or with a reactive derivative thereof, or cyclising a compound of the formula

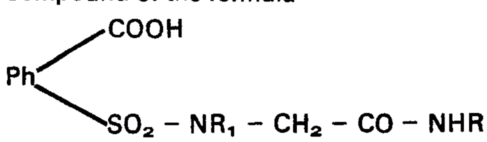

(IV)

or a functional carboxy derivative and/or a salt thereof to produce a compound of the formula I, or subjecting a compound of the formula

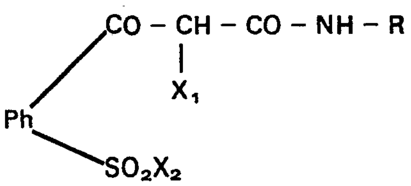

(V)

wherein one of $X_1$ and $X_2$ is a group of the formula $-NHR_1$ and the other is a free or etherified or reactive esterified hydroxyl group or amino group X, to intramolecular cyclisation, or in a compound of the formula

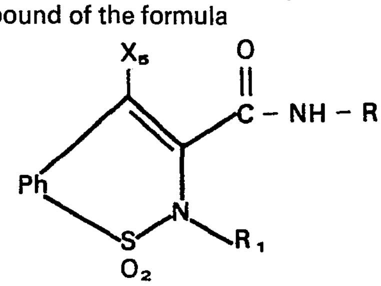

(VI)

wherein $X_5$ is a group which can be converted into hydroxyl, or in a salt thereof, converting $X_5$ into a hydroxyl group, or in a compound of the formula VII

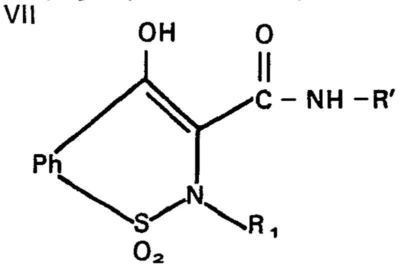

(VII)

wherein R' is a radical which can be cyclised to the substituted or unsubstituted benzopyrone radical R, or in a salt thereof, cyclising R' to R, and, if desired, converting a resultant salt into the free compound or into another salt, or converting a resultant salt into the free compound.

## Revendications

1. 3,4-Dihydro-2H-1,2-benzothiazin-1,1-dioxyde substitué de formule I

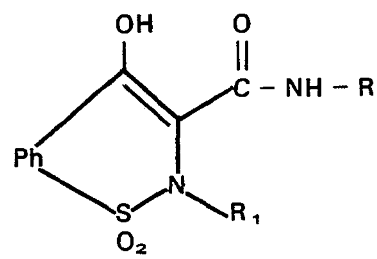

(I)

où Ph représente un radical 1,2-phénylène éventuellement substitué par un alcoyle inférieur, un alcoxy inférieur, un alcanoyle inférieur, un halogène, un nitro et/ou un trifluorométhyl, R représente un radical benzopyrone éventuellement substitué par des radicaux alcoyle inférieur, alcoxy inférieur, halogène, alcanoyloxy inférieur, hydroxy, benzoyloxy, un amino éventuellement substitué par un alcoyle inférieur ou un alcanoyle inférieur ou, sur deux atomes de carbone voisins, par un alcoylène inférieur ou un alcoylène inférieur-dioxy, et $R_1$ représente un hydrogène, un alcoyle inférieur, un alcényle inférieur, un hydroxyalcoyle inférieur, un alcoxy inférieur-alcoyle inférieur ou un radical phénylalcoyle inférieur, où, dans les groupes mentionnés, le phényle peut être substitué par un alcoyle inférieur, un alcoxy inférieur, un halogène, un trifluorométhyle et/ou un nitro.

2. Composé selon la revendication 1 où Ph représente un 1,2-phénylène non substitué ou substitué par un alcoyle inférieur ayant jusqu'à 4 atomes de carbone, un alcoxy inférieur ayant jusqu'à 4 atomes de carbone, un halogène dont le numéro atomique va jusqu'à 35, un nitro ou un trifluorométhyle, R représente un radical 2-oxo-2H-1-benzopyrannyle lié en position 3, 4, 6 ou 7 ou un radical 4-oxo-4H-1-benzopyrannyle lié en position 3, 6 ou 7, qui peut être non substitué ou substitué par un alcoyle inférieur ayant jusqu'à

4 atomes de carbone, un alcoxy inférieur ayant jusqu'à 4 atomes de carbone, un halogène dont le numéro atomique va jusqu'à 35, un alcanoyloxy inférieur ayant jusqu'à 4 atomes de carbone, un hydroxy, un amino, un N-mono- ou N,N-dialcoyle inférieur-amino ayant jusqu'à 4 atomes de carbone dans la partie alcoyle, un alcanoyle inférieur-amino ayant jusqu'à 4 atomes de carbone et/ou sur deux atomes de carbone voisins par un alcoylène inférieur ou un alcoylène inférieur-dioxy ayant jusqu'à 4 atomes de carbone, et $R_1$ représente un hydrogène ou un alcoyle inférieur ayant jusqu'à 4 atomes de carbone, ou un de ses sels formés avec une base.

3. Composé selon la revendication 1 de formules Ib et Ic

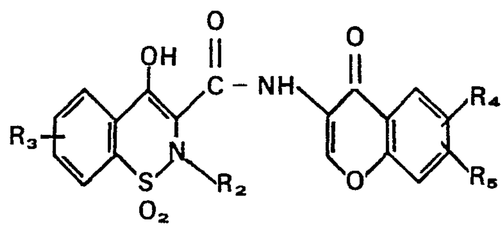

(Ib)

et

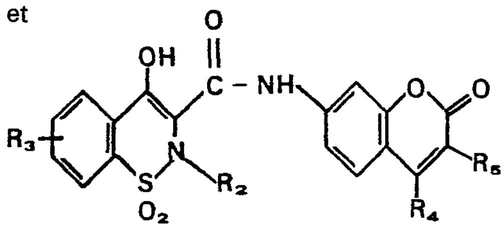

(Ic)

où $R_2$ représente un hydrogène ou un alcoyle inférieur ayant jusqu'à 4 atomes de carbone, $R_3$ représente un hydrogène, un alcoyle inférieur ayant jusqu'à 4 atomes de carbone, un alcoxy inférieur ayant jusqu'à 4 atomes de carbone, un halogène dont le numéro atomique peut aller jusqu'à 35, un trifluorométhyle ou un nitro, et $R_4$ et $R_5$ représentent indépendamment l'un de l'autre un hydrogène, un alcoyle inférieur ayant jusqu'à 4 atomes de carbone, ou un alcoxy inférieur ayant jusqu'à 4 atomes de carbone, ou en commun un radical alcoylène inférieur ou alcoylène inférieur-dioxy à 3 ou 4 chaînons liés sur des atomes de carbone voisins ayant jusqu'à 4 atomes de carbone, ou, dans la formule Ic, $R_4$ représente un hydroxy et $R_5$ un hydrogène ou un alcoyle inférieur ayant jusqu'à 4 atomes de carbone, ou un de ses sels formés avec une base.

4. Composé selon la revendication 3, où $R_2$ représente un alcoyle inférieur ayant jusqu'à 4 atomes de carbone, $R_3$ représente un hydrogène, et $R_4$ et $R_5$ représentent, indépendamment l'un de l'autre, un hydrogène, un alcoyle inférieur ou alcoxy inférieur ayant jusqu'à 4 atomes de carbone, ou un de ses sels formés avec une base.

5. Composé du groupe constitué par le
2-Méthyl-3,4-dihydro-4-oxo-N-(4-oxo-4H-1-benzopyran-3-yl)-2H-1,2-benzothiazin-3-carboxamide-1,1-dioxide
et ses sels formés avec une base.

6. Composé selon la revendication 1 constitué de

2-Méthyl-3,4-dihydro-4-oxo-N-(2-oxo-4-méthyl-2H-1-benzopyran-7-yl)-2H-1,2-benzothiazin-3-carboxamide-1,1-dioxide,

2-Méthyl-3,4-dihydro-4-oxo-N-(4-oxo-4,6,7,8-tétrahydro-cyclopenta-[g]-1-benzopyran-3-yl)-2H-1,2-benzothiazin-3-carboxamide-1,1-dioxide,

2-Méthyl-3,4-dihydro-4-oxo-N-(2-oxo-4-hydroxy-6-méthyl-2H-1-benzopyran-3-yl)-2H-1,2-benzothiazin-3-carboxamide-1,1-dioxyde,

2-Méthyl-3,4-dihydro-4-oxo-N-(4-oxo-6,7-diméthyl-4H-1-benzopyran-3-yl)-2H-1,2-benzothiazin-3-carboxamide-1,1-dioxyde,

2-Méthyl-3,4-dihydro-4-oxo-N-(4-oxo-6-méthoxy-4H-1-benzopyran-3-yl)-2H-1,2-benzothiazin-3-carboxamide-1,1-dioxyde,

2-Méthyl-3,4-dihydro-4-oxo-N-(4-oxo-5,7-diméthyl-4H-1-benzopyran-3-yl)-2H-1,2-benzothiazin-3-carboxamide-1,1-dioxyde,

2-Méthyl-3,4-dihydro-4-oxo-N-(4-oxo-8-méthyl-4H-1-benzopyran-3-yl)-2H-1,2-benzothiazin-1,1-dioxyde,

2-Méthyl-3,4-dihydro-4-oxo-N-(4-oxo-6-méthyl-4H-1-benzopyran-3-yl)-2H-1,2-benzothiazin-3-carboxamide-1,1-dioxyde,

2-Méthyl-3,4-dihydro-4-oxo-N-(2-oxo-4-hydroxy-6-chloro-2H-1-benzopyran-3-yl)-2H-1,2-benzothiazin-3-carboxamide-1,1-dioxyde,

2-Méthyl-3,4-dihydro-4-oxo-N-(2-oxo-4-hydroxy-6,8-dichloro-2H-1-benzopyran-3-yl)-2H-1,2-benzothiazin-3-carboxamide-1,1-dioxyde,

2-Méthyl-3,4-dihydro-4-oxo-N-(2-oxo-4-hydroxy-4,6,7,8-tétrahydrocyclopenta-[g]-2H-1-benzopyran-3-yl)-2H-1,2-benzothiazin-3-carboxamide-1,1-dioxyde,

2-Méthyl-3,4-dihydro-4-oxo-N-(2-oxo-4-hydroxy-8-méthyl-2H-1-benzopyran-3-yl)-2H-1,2-benzothiazin-3-carboxamide-1,1-dioxyde,

2-Méthyl-3,4-dihydro-4-oxo-N-(4-oxo-6-chloro-8-méthyl-4H-1-benzopyran-3-yl)-2H-1,2-benzothiazin-3-carboxamide-1,1-dioxyde,

3,4-Dihydro-4-oxo-N-(4-oxo-4H-1-benzopyran-3-yl)-2H-1,2-benzothiazin-3-carboxamide-1,1-dioxyde,

2-Ethyl-3,4-dihydro-4-oxo-N-(4-oxo-4H-1-benzopyran-3-yl)-2H-1,2-benzothiazin-3-carboxamide-1,1-dioxyde, et

2-Allyl-3,4-dihydro-4-oxo-N-(4-oxo-4H-1-benzopyran-3-yl)-2H-1,2-benzothiazin-3-carboxamide-1,1-dioxyde,

ou de leurs sels.

7. Composé selon l'une des revendications 1 à 6, sous la forme du sel de sodium ou du sel de zinc.

8. Composé selon l'une des revendications 1 à 7, à action anti-inflammatoire.

9. Préparations pharmaceutiques contenant un composé selon l'une des revendications 1 à 8, éventuellement sous la forme d'un sel pharmaceutiquement acceptable formé avec une base, et avec des additifs et/ou supports habituels.

10. Composé selon l'une des revendications 1 à 8, éventuellement sous la forme d'un sel pharmaceutiquement acceptable formé avec une base, aux fins de traitement thérapeutique du corps humain ou animal.

11. Composé selon l'une des revendications 1 à 8 aux fins d'application selon la revendication 9 comme anti-inflammatoire.

12. Application d'un composé selon l'une des revendications 1 à 8 à la préparation d'un médicament, en particulier d'un anti-inflammatoire.

13. Composé selon l'une des revendications 1 à 8 aux fins de lutte contre les maladies, en particulier les maladies inflammatoires.

14. Procédé de préparation de nouveaux 3,4-dihydro-2H-1,2-benzothiazin-1,1-dioxydes de formule I

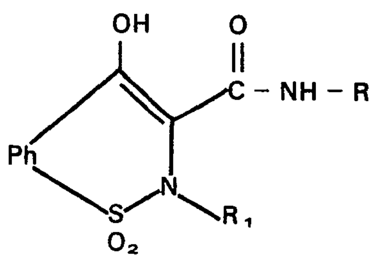

(I)

où Ph, R et $R_1$ sont tels que définis dans la revendication 1, et de leurs sels, caractérisé en ce qu'on fait réagir un composé de formule

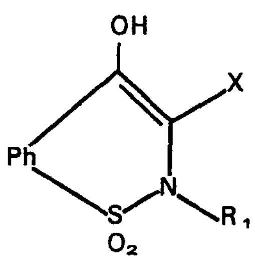

(II)

où X représente un groupe carboxy éventuellement fonctionnellement modifié, ou un de ses sels, avec un composé de formule $R-NH_2$ (III) ou avec un de ses dérivés réactifs, ou en ce qu'on cyclise un composé de formule

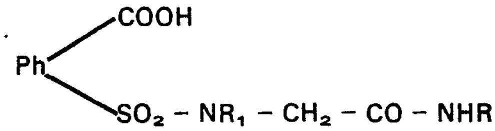

(IV)

ou un de ses dérivés carboxy fonctionnels et/ou sels pour donner un composé de formule I, ou en ce qu'on cyclise de façon intramoléculaire un composé de formule

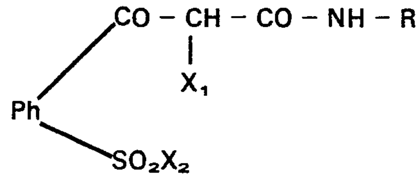

(V)

où l'un des radicaux $X_1$ et $X_2$ représente un groupe de formule $-NHR_1$ et l'autre un groupe hydroxy ou un groupe amino éventuellement éthérifié ou estérifié de façon réactive, ou en ce qu'on transforme, dans un composé de formule (VI)

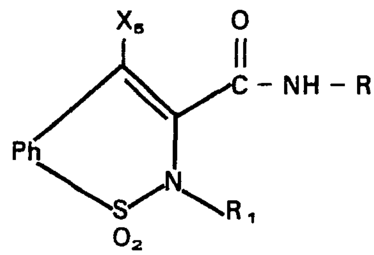

(VI)

où $X_5$ représente un groupe transformable en un hydroxy, ou dans un de ses sels, $X_5$ en hydroxy, ou en ce qu'on cyclise dans un composé de formule VII

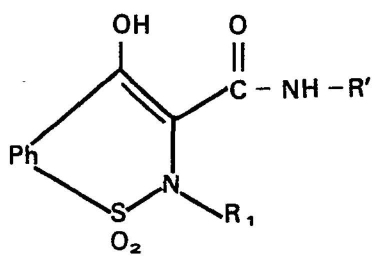

(VII)

où R' représente un radical cyclisable pour donner le radical benzopyrone R éventuellement substitué, ou dans un de ses sels, R' en R, et, si on le désire, en ce qu'on transforme un sel obtenu selon le procédé en le composé libre ou en un autre sel ou un sel obtenu selon le procédé en le composé libre.